# EUROPEAN PATENT SPECIFICATION

(11) **EP 0 690 715 B1**
(45) Date of publication and mention of the grant of the patent: **28.05.2003**
(21) Application number: 94911001.9
(22) Date of filing: 28.03.1994
(51) Int. Cl.: A61K 31/505, A61F 2/00, A61F 13/00, A61K 31/445, A61P 37/00

(54) **TOPICAL AND SYSTEMIC APPLICATION OF BUSPIRONE OR DERIVATIVES THEREOF FOR TREATMENT OF PATHOLOGICAL CONDITIONS ASSOCIATED WITH IMMUNE RESPONSES**
TOPISCHE UND SYSTEMISCHE ANWENDUNG VON BUSPIRON UND SEINEN DERIVATEN ZUR BEHANDLUNG VON PATHOLOGISCHEN ZUSTÄNDEN, DIE MIT IMMUNANTWORTEN VERBUNDEN SIND
UTILISATION TOPIQUE OU SYSTEMIQUE DE BUSPIRONE OU DE DERIVES DE CELLE-CI POUR LE TRAITEMENT DE TROUBLES PATHOLOGIQUES ASSOCIES A DES REACTIONS IMMUNITAIRES

(30) Priority: 26.03.1993 US 37225; 26.03.1993 US 37271
(43) Date of publication of application: 10.01.1996
(73) Proprietor: Beth Israel Hospital Association, Boston, Massachusetts 02215 (US)
(72) Inventor: SHARPE, Richard J., Newtonville, MA 02160 (US); ARNDT, Kenneth A., Newton Centre, MA 02159 (US); GALLI, Stephen J., Winchester, MA 01890 (US); MELTZER, Peter C., Lexington, MA 02173 (US); RAZDAN, Raj K., Belmont, MA 02178 (US); SARD, Howard P., Arlington, MA 02174 (US)
(74) Representative: Bassett, Richard Simon
(86) International application number: US9403353
(87) International publication number: WO94022448

(56) References cited:
- WO-A-91/02527
- WO-A-91/13622
- WO-A-93/24144
- US-A- 4 963 557

## Description

### Background of the Invention

This application is in the area of the topical and systemic administration of buspirone or derivatives thereof for the treatment of pathological conditions associated with immune responses.

The immune system specifically recognizes and selectively eliminates foreign invaders, or other antigenic agents, by a process known as the immune response. The immune response has three major characteristics: it responds adaptively to foreign invaders, it exhibits strong specificity, and it displays a long-term memory of earlier contacts with specific foreign pathogens or antigens. The immune response involves the production of antibodies and/or the destruction of antigenic cells by T lymphocytes; both the antibodies and the T lymphocytes are highly specific for the antigen or hapten.

When directed against an infectious organism, the immune response can provide great benefit to the host. As an example, an important component of current public health practices is the use of vaccines to elicit immune responses against infectious organisms that cause severe illness and death. However, when directed against agents that are relatively innocuous, such as pollen, animal dander, and certain plant resins, the cells, antibodies, and mediators which represent the effector components of the immune response can cause damage to the host's tissues that is out of proportion to any threat to health posed by the antigenic agent that first elicited the response. Topical disorders that involve the immune system can result in result in one or more of the following symptoms or signs: itching, swelling, redness, blisters, crusting, ulceration, pain, scaling, cracking, hair loss, scarring, or oozing of fluid involving the skin, eye, or mucosal membranes.

Cutaneous contact hypersensitivity responses are complex expressions of cellular immunity characterized by antigen-dependent changes in lymphocyte traffic, the recruitment of circulating leukocytes to the site of antigen challenge (leukocyte infiltration), and alterations in vascular permeability and blood flow resulting in tissue swelling (edema). While T cells are required for the expression and immunological specificity of the response, many other cell types also have roles in the reaction, including Langerhans' cells, keratinocytes, and vascular endothelial cells. Antigen presentation is thought to be effected primarily by Langerhans' cells, whereas much of the local expression of the response is thought to be regulated by cytokines derived from both T cells and accessory cells. Pharmacological studies have indicated that a number of mediators in addition to cytokines may contribute to the expression of contact hypersensitivity and other forms of cell-mediated immunity. There has been particular interest in the role of serotonin (5-hydroxytryptamine, 5-HT) in these reactions. For example, serotonin has been shown to have a wide range of actions on T cells and other effector cells *in vitro* or *in vivo*, and pharmacological agents that deplete or antagonize serotonin can diminish expression of cell-mediated immunity. Early studies raised the possibility that such agents might reduce cell-mediated immunity by antagonizing or depleting mast cell-associated serotonin. However, more recent findings indicate that at least one of these drugs, reserpine, can inhibit contact hypersensitivity independently of mast cells, probably through direct effects on T cells.

In humans and companion animals, cutaneous contact hypersensitivity responses can occur on exposure to certain plant resins, such as those of poison ivy, and other commonly encountered agents in the environment. In individuals sensitized to such commonly encountered agents, a severe contact reaction can result upon exposure, with significant associated morbidity. Severe or repeated contact hypersensitivity reactions can be followed by significant chronic changes, such as scarring of affected tissues, itchiness, swelling, scaling and oozing of tissue fluid through the skin surface. This pathology may predispose the patient to bacterial superinfection. In the eye, chronic immune responses can lead to diminished vision or actual blindness. In the lung, chronic immune responses, such as chronic allergic asthma, can result in serious chronic lung disease.

Examples of pathological or undesired immune responses other than cutaneous contact hypersensitivity, including systemic disorders, include but are not limited to host rejection of foreign organ or tissue transplants; graft-vs-host disease in which donor immunological cells present in the graft attack host tissues in the recipient of the graft; diseases with proven or possible autoimmune components, such as rheumatoid arthritis and juvenile rheumatoid arthritis, aphthous ulcer, lichen planus, psoriatic arthritis, psoriasis, excema, conjunctivitis, Sjogren's Syndrome, including keratoconjunctivitis sicca secondary to Sjogren's Syndrome, iritis, alopecia areata, cutaneous lupus erythematosus, sclerodenna, vaginitis, proctitis, drug eruptions, leprosy reversal reactions, erythema nodosum leprosum, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener's granulomatosis, chronic active hepatitis, myasthenia gravis, Stevens-Johnson syndrome, idiopathic sprue, Crohn's disease, ulcerative colitis, Graves ophthalmopathy, sarcoidosis, primary biliary cirrhosis, primary juvenile diabetes, uveitis posterior, and interstitial lung fibrosis; asthma; allergic asthma, allergic responses due to arthropod bite reactions, and inappropriate allergic responses to other environmental stimuli such as atopic dermatitis and hypersensitivity to pollen, insect stings and certain foods.

Various therapeutics have been utilized as immunosuppressants including steroid hormones, antiproliferatives such as methotrexate and azathioprine, cyclosporine, alkylating agents such as cyclophosamide and busulfan, psoralen plus ultraviolet A (PUVA), and antibiotics. Corticosteroids, when administered systemically, can be effective but can be associated with significant and potentially dangerous side effects. Topically applied corticosteroids have some efficacy in treating these conditions, but are only partially effective in many instances and have their own significant side effects, including atrophy of tissue, formation of telangiectasia, blanching, and a myriad of systemic effects if significantly absorbed. As a result, there still remains a strong need to provide new immunosuppressive agents that can minimize or prevent pathological immune responses.

In contrast to the immune response, an inflammatory response is a pathologic condition that can occur in response to immunologically non-specific injury, either from physical (such as trauma), chemical, or biological agents. An inflammatory response is characterized by increased blood flow and redness in the inflamed area, increased capillary permeability and edema, and recruitment of immunologically non-specific white blood cells, especially neutrophils, that remove injurious material and promote repair. Unlike immune responses, inflammatory responses do not respond adaptively to the inciting stimulus, do not show specificity and do not exhibit long term memory. Cellular products of lymphocytes may contribute to or induce an inflammatory response. However, because of the differences in mechanisms, a compound can function as an anti-inflammatory agent without having immunosuppressive properties. Phenylbutazone, indomethacin, aspirin, ibuprofen, and acetaminophen are examples of anti-inflammatory compounds which have no significant immunosuppressive activity, as demonstrated by their lack of a significant effect on immunologically mediated responses, such as contact hypersensitivity.

PCT International Publication No. WO 91/02527 discloses a method and composition to treat cutaneous, mucosal, or ocular hypersensitivity that includes administering an effective amount of reserpine, spiperone, or other serotonin antagonist.

It is an object of the present invention to present a use of a compound in the manufacture of a medicament for the topical treatment of cutaneous, mucosal and ocular pathologies associated with immune responses.

It is yet another object of the present invention to present a use of a compound in the manufacture of a medicament for the systemic treatment of pathogenic conditions associated with immune responses.

### Summary of the Invention

The invention provides a use of a compound as defined in Claim 1 in the manufacture of a medicament for treating a cutaneous, occular or mucusal pathology associated with an immune response wherein the medicament is for topical application.

The compounds are useful as topical agents in treating contact dermatitis, atopic dermatitis, eczematous dermatitis, psoriasis, Sjogren's Syndrome, including keratoconjunctivitis sicca secondary to Sjogren's Syndrome, alopecia areata, allergic responses due to arthropod bite reactions, Crohn's disease, aphthous ulcer, iritis, conjunctivitis, keratoconjunctivitis, ulcerative colitis, asthma, allergic asthma, cutaneous lupus erythematosus, scleroderma, vaginitis, proctitis, and drug eruptions. The compounds may also be useful in reducing the infiltration of skin by malignant leukocytes in diseases such as mycosis fungoides. These compounds are also effective when used in a medicament for treating an aqueous-deficient dry eye state (such as immune mediated keratoconjunctivitis) in a patient suffering therefrom, by administering the compound topically to the eye.

The invention also provides a use of a compound as defined in Claim 1 in the manufacture of a medicament for treating a mammal in need of immunosuppression, wherein the medicament is for systemic administration.

The parent buspirone can have a neuroleptic effect when administered systemically (but not typically when administered topically), however, it is a model of an active immunosuppressant. Derivatives of buspirone are considered to be immunosuppressants if they suppress the ear swelling associated with an experimental contact hypersensitivity response by at least 40% at 24 hours after specific antigen challenge.

### Brief Description of the Figures

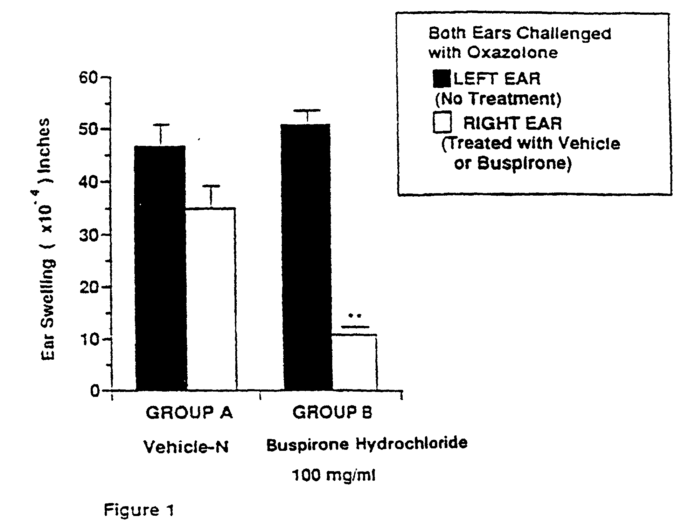

Figure 1 - Effect of topically administered buspirone HCl on tissue swelling associated with oxazolone-induced contact hypersensitivity reactions. Oxazolone (10 µl of a 0.5% (w:w) solution) was applied to both ears of all mice and the change in ear thickness was measured at a specified interval thereafter. Buspirone HCl (100 mg/ml) (Group B) or vehicle alone (Group A) was applied to the right ear of Balb/c mice 2 hours after challenge. The change in ear thickness (post-challenge value minus baseline pre-challenge value) was measured 24 hours after oxazolone challenge. The data are presented as the mean ± SEM (standard error of the mean). The reduction in ear swelling observed with 100 mg/ml buspirone HCl was significant when compared to the reactions observed in the vehicle treated animals (Group A) (**=*p*<0.01).

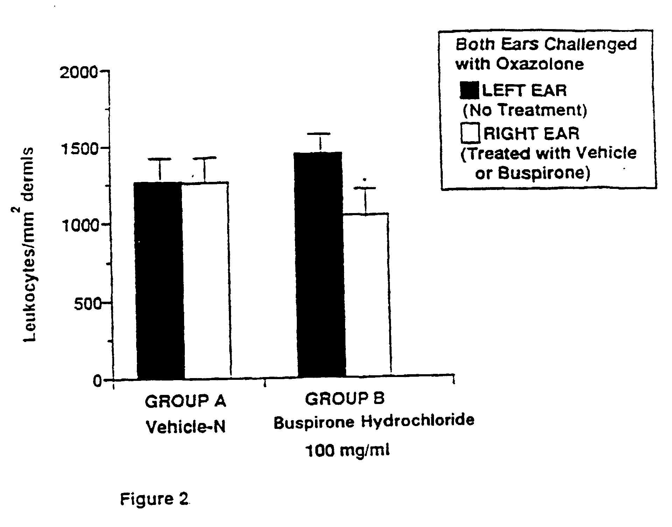

Figure 2 - Effects of topical treatment with 100 mg/ml buspirone HCl on leukocyte infiltration associated with 24-hour contact hypersensitivity reactions. These data (mean ± SEM) are derived from the same mice whose ear thickness values are shown in Figure 1. The reduction in leukocyte infiltration observed in animals treated with 100 mg/kg buspirone HCl was significant when compared to the reactions observed in animals treated with vehicle alone (* =*p*<0.05).

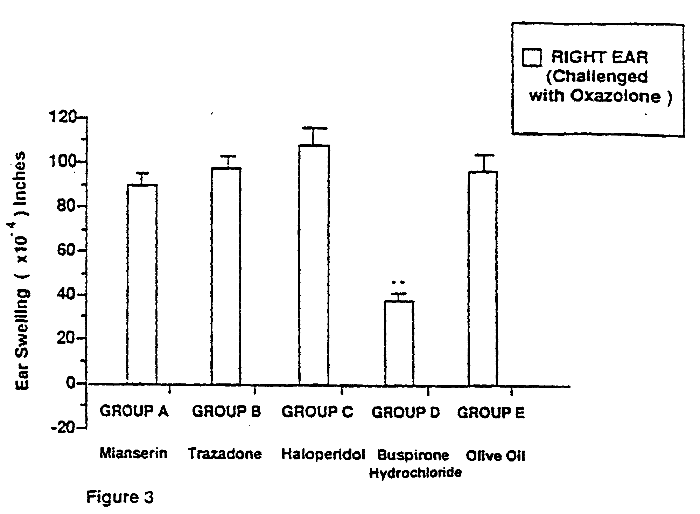

Figure 3 - Comparative effects of 50 mg/kg subcutaneous administration of mianserin HCl (Group A), trazadone HCl (Group B), haloperidol (Group C), buspirone HCl (Group D), and vehicle (Group E) on the tissue swelling associated with oxazolone-induced cutaneous contact hypersensitivity reactions. Buspirone HCl, the other agents, or vehicle alone were administered to BALB/c mice 1 hour after right ears only were challenge for contact hypersensitivity. The change in ear thickness (post-challenged value minus baseline pre-challenge value) was measured 24 hours after oxazolone challenge. The data are presented as the mean ± SEM. The reduction in ear swelling observed with buspirone HCl was significant when compared to the reactions observed in the challenged right ears of the control, vehicle (Group E, olive oil) treated animals (**=*p*<0.01), whereas haloperidol, trazadone and mianserin did not significantly suppress the tissue swelling associated with contact hypersensitivity.

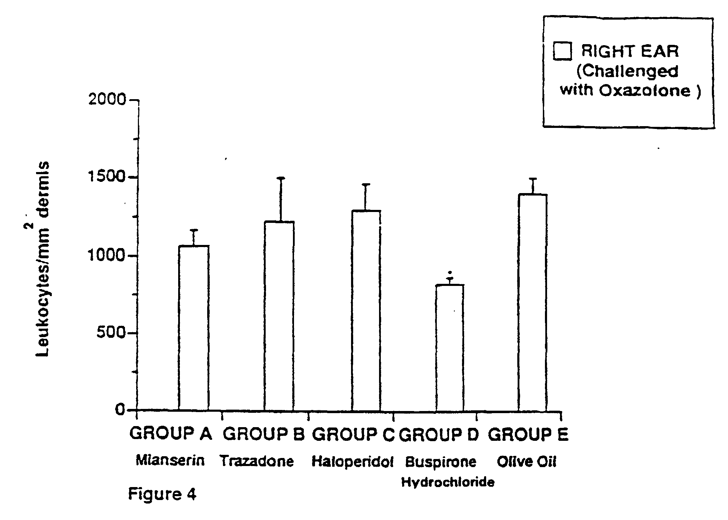

Figure 4 - Comparative effects of subcutaneous administration of 50 mg/kg mianserin HCl (Group A), trazadone HCl (Group B), haloperidol (Group C), buspirone HCl (Group D), and systemic vehicle (Group E) on leukocyte infiltration associated with 24-hour contact hypersensitivity reactions. These data (mean ± SEM) are derived from the same mice whose ear thickness values are shown in Figure 3. The reduction in leukocyte infiltration observed in the right (oxazolone-challenged) ears of animals treated with buspirone HCl was significant when compared to the reactions observed in animals treated with vehicle alone (*=*p*<0.05), while haloperidol, trazadone and mianserin did not significantly suppress the leukocyte infiltration associated with contact hypersensitivity.

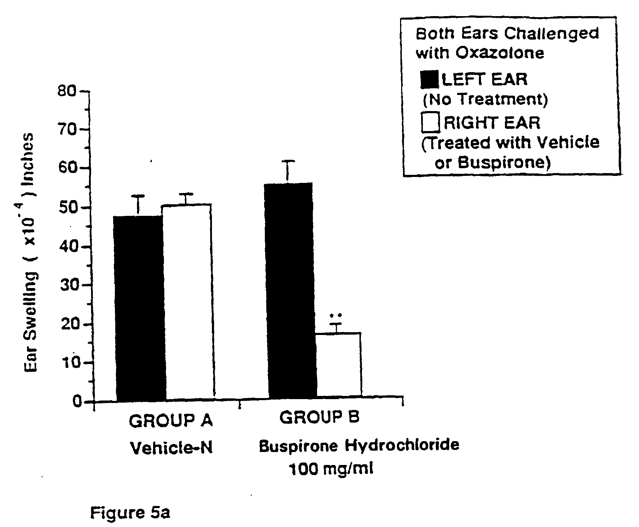

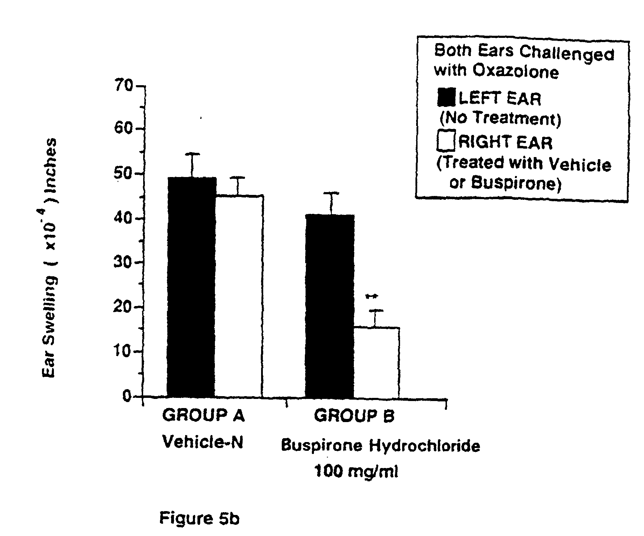

Figure 5a,b - Effect of topically administered buspirone HCl on tissue swelling associated with oxazolone-induced contact hypersensitivity reactions. Oxazolone was applied to both ears of all mice at different times either pre- or post-buspirone HCl treatment and the change in ear thickness was measured at a specified interval thereafter. a. Two hours before oxazolone challenge, 100 mg/ml buspirone HCl in Vehicle-N was applied to both surfaces of the right ears of Group A mice, whereas vehicle alone (0% buspirone HCl) was applied to both surfaces of the right ears of the control, vehicle only (Group B Vehicle-N) animals. The ears were measured 24 hours after oxazolone challenge. Local prechallenge treatment of the right ear with buspirone HCl significantly suppressed tissue swelling in the treated ear (***p*<0.01 vs contralateral oxazolone treated ears or vs right ears of vehicle treated group). Treatment of the right ear with 100 mg/ml buspirone HCl had no significant effect on the magnitude of swelling in the contralateral oxazolone treated ear. b. In a separate experiment, twenty-four hours after oxazolone challenge, 100 mg/ml buspirone HCl in Vehicle-N was applied to both surfaces of the right ears of Group B mice, whereas vehicle alone (0% buspirone HCl) was applied to both surfaces of the ears of control vehicle only (Group A vehicle-N) mice. The change in ear thickness was determined 24 hours after treatment with buspirone HCl, i.e. at 48 hours after challenge with oxazolone. Treatment with buspirone HCl significantly diminished contact hypersensitivity reactions in the right ears of the treated animals (**=*p*<0.01 when compared to the right ears in the control mice (Group A), and *p*<0.05 when compared to the contralateral ears of the same mice). The reactions in the left ears of the mice treated on the right ears with buspirone HCl (Group B) were not reduced when compared to reactions in the left ears of the vehicle-treated mice (Group A).

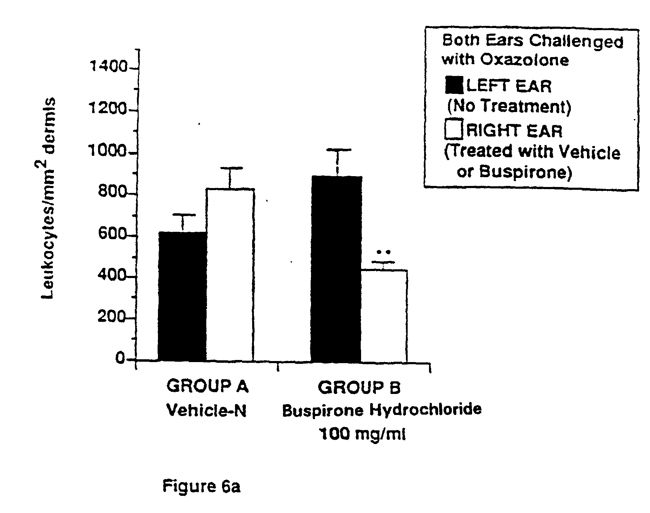

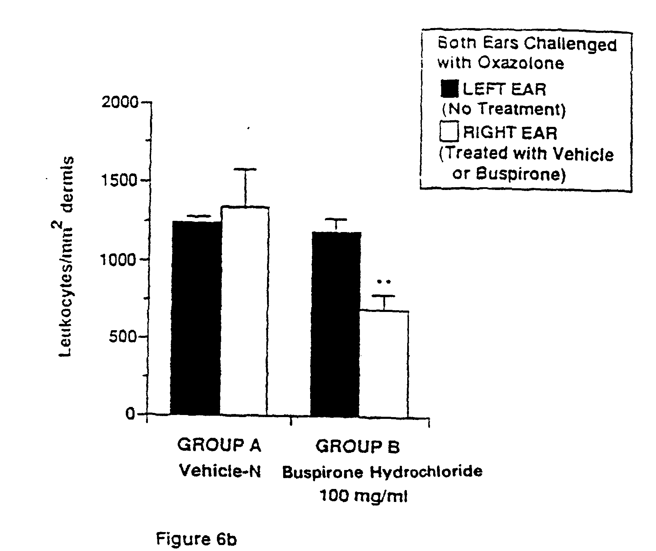

Figure 6a,b - Effect of topical treatment with buspirone HCl on leukocyte infiltration associated with oxazolone-induced contact hypersensitivity reactions. These data (mean ± SEM) are from the same mice whose ear thickness measurements are presented in Figure 5 a,b. Biopsies were performed 24 hours (a), or 48 hours (b) after application of oxazolone. Topical prechallenge treatment with buspirone HCl significantly diminished the reactions when compared to those in vehicle-treated mice (6a), as did topical postchallenge treatment with buspirone HCl (6b) (**=*p*<0.01 in both 6a and b).

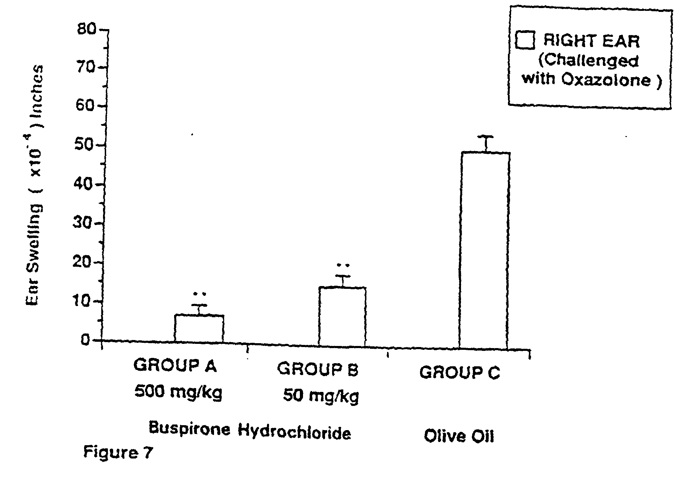

Figure 7 - Effect of systemic buspirone HCl (Group A, 500 mg/kg; Group B, 50 mg/kg) administered subcutaneously on tissue swelling associated with oxazolone induced cutaneous hypersensitivity reactions of the right ear. Buspirone HCl (Groups A and B) or vehicle alone (Group C) was administered to Balb/c mice 1 hour after challenge. Change in ear thickness (post-challenge - baseline pre-challenge value) was measured 24 hours after oxazolone challenge. The data are presented as ±SEM. Systemic treatment with buspirone HCl (at 500 or 50 mg/kg) significantly reduced the tissue swelling associated with contact hypersensitivity compared to the responses observed in the right ears of mice treated with vehicle alone (Group C) (**=p<0.01).

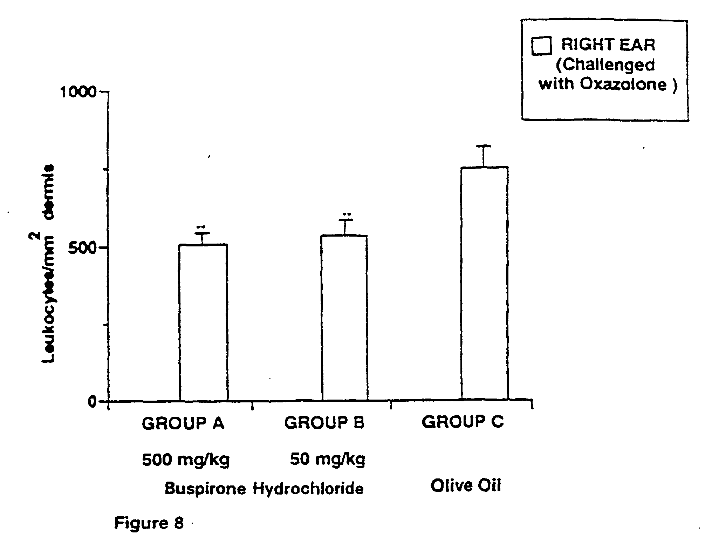

Figure 8 - Effect of systemic buspirone HCl (500 or 50 mg/kg buspirone, administered subcutaneously) on leukocyte infiltration associated with oxazolone induced cutaneous hypersensitivity reactions of the right ear. These data are derived from the same mice whose ear thickness values are shown in Figure 7. Systemic treatment with buspirone HCl (at 500 or 50 mg/kg) significantly reduced the leukocyte infiltration when compared to the reaction observed in animals treated with vehicle alone (**=p<0.01 for both Group A and Group B).

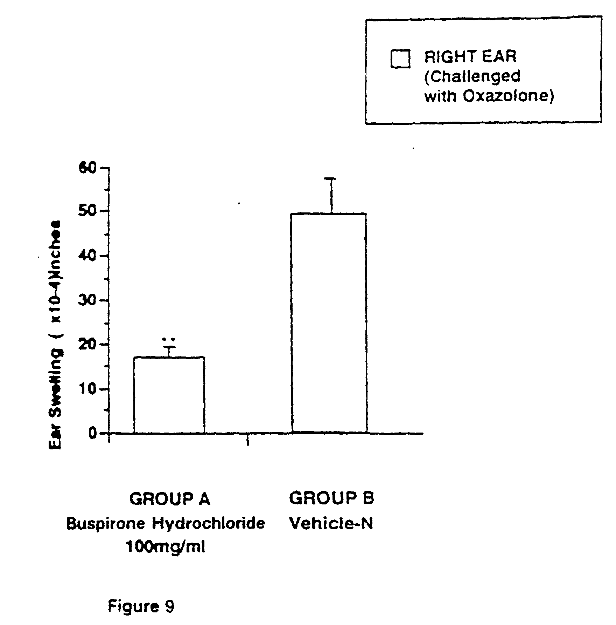

Figure 9 - The effect of topical treatment with buspirone HCl on suppression of the sensitization phase of oxazolone challenge. Buspirone HCl at 100 mg/ml (Group A) or vehicle-N (Group B) was applied to abdomen of mice 3 days prior to sensitization of Balb/c mice with 4% oxazolone. This treatment was repeated 3 days after sensitization. The right ears of all mice were then challenged with 0.5% oxazolone. The change in the ear thickness was measured 24 hours after oxazolone challenge. The data are presented as the mean ± SEM. The reduction in ear swelling observed with buspirone HCl treatment (Group A) was significant when compared to those treated with vehicle only (Group B). (**=p<0.01).

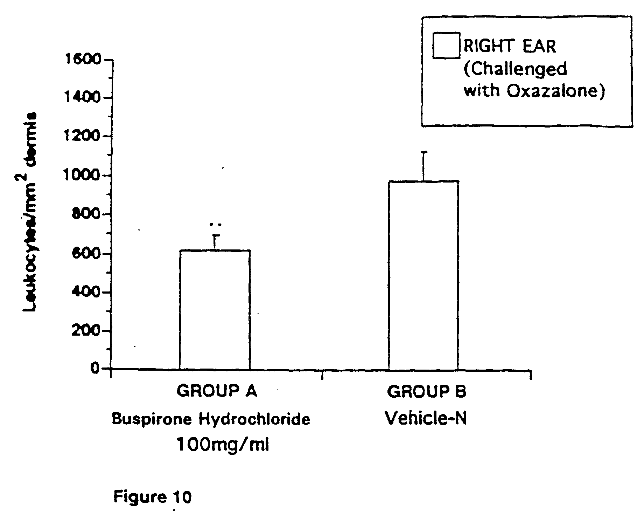

Figure 10 - Effect of topical treatment with buspirone HCl (100 mg/ml) on leukocyte infiltration associated with suppression of sensitization phase of oxazolone challenge. These data are from the same mice whose ear thickness measurements are presented in Fig. 9. Biopsies were performed 24 hours after oxazolone challenge. Topical buspirone HCl, administered both pre- and post-sensitization (Group A), significantly diminished the reactions as compared to the vehicle-only treated mice (Group B). (**=p<0.01).

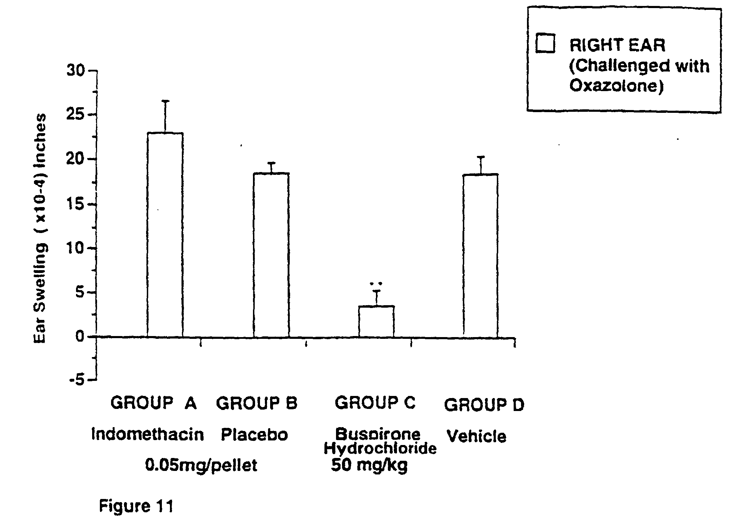

Figure 11 - Effect of systemic buspirone HCl treatment (50 mg/kg, administered subcutaneously), indomethacin, or placebo pellets (0.05 mg/pellet, implanted subcutaneously) on oxazolone induced contact hypersensitivity. Four groups of mice were sensitized to oxazolone by treatment with 4% oxazolone. Three days later two groups were implanted with 0.05 mg indomethacin (Group A) and placebo pellets (Group B). Three days later, right ears of mice in all four groups were challenged with 0.5% oxazolone. One hour post challenge, remaining two groups were treated with buspirone HCl at 50 mg/kg (Group C) or vehicle (Group D). Ear swelling was measured 24 hours after oxazolone challenge. Pre-challenge treatment with indomethacin or placebo (Groups A and B, respectively) did not have a significant effect on the hypersensitivity response as compared to control (Group D) in which vehicle alone was administered post-challenge. However, buspirone HCl treatment (Group C), applied post-challenge, significantly reduced ear swelling as compared to the control group (Group D, vehicle only). (**=p<0.01).

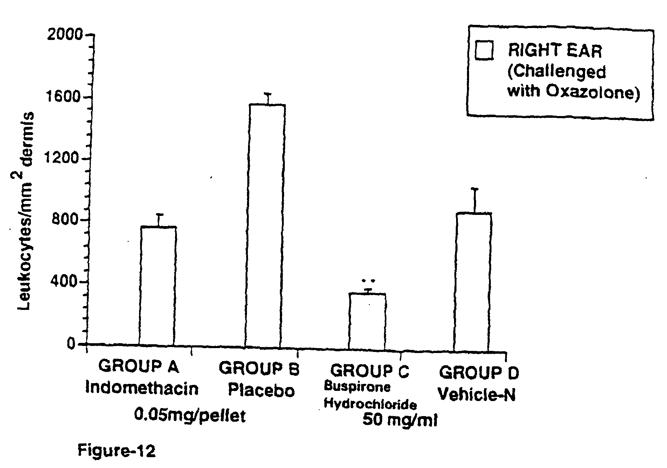

Figure 12 - Effect of systemic buspirone HCl treatment (Group C), indomethacin or placebo treatment (Groups A and B, respectively) on leukocyte infiltration associated with oxazolone induced cutaneous hypersensitivity reaction. These data are from the same mice whose ear thickness measurements are presented in Fig. 11. Biopsies were performed 24 hours after oxazolone challenge. Buspirone HCl treatment (Group C) significantly reduced leukocyte infiltration as compared to the control group (Group D, vehicle only) (**=p<0.01); whereas indomethacin and placebo treatment had no significant effect, when compared to control groups.

### Detailed Description of the Invention

The term alkyl, as used herein, unless otherwise specified, refers to a saturated straight, branched, or cyclic hydrocarbon of C₁ to C₂₀, including methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl.

The term aryl, as used herein, and unless otherwise specified, refers to phenyl or substituted phenyl, wherein the substituent is independently halo, alkyl, or oxy(alkyl) (for example, methyoxy, ethoxy, etc.), and wherein the aryl can have up to three substituents.

The term heterocycle refers to a cyclic moiety that has O, S, or N in the aromatic ring, including but not limited to, pyrryl, furyl, pyridyl, thiophene, pyrimidyl, thienyl, isothiazolyl, imidazolyl, tetrazolyl, pyrazinyl, pyrimidyl, quinolyl, isoquinolyl, benzothienyl, isobenzofuryl, pyrazolyl, indolyl, purinyl, carbozolyl, and isoxazolyl and the like, optionally substituted with halo (Cl, Br, I, or F), alkyl, oxyalkyl, aryl or oxyaryl.

The term aralkyl refers to an aryl group with an alkyl substituent.

The term alkaryl refers to an alkyl group that has an aryl substituent.

The term alkene, as referred to herein, and unless otherwise specified, refers to an alkene group of C₂ to C₁₀, and specifically includes vinyl, and allyl.

### I. Structure and Synthesis of Buspirone Derivatives

The parent buspirone is 8-[4-[4-(2-pyrimidinyl)-1-piperaziny]butyl]-8-azaspiro-[4.5]decane-7,9-dione, which has the structure illustrated below.

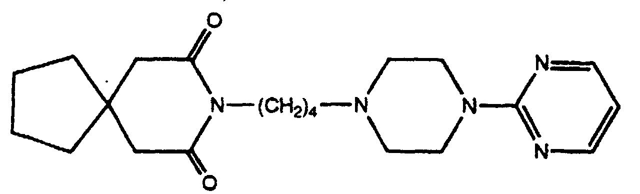

The term "buspirone derivative" as used herein refers to a compound that exhibits an immunosuppressive effect, for example, as measured using the assay set out in Example 1, i.e., it suppresses the ear swelling associated with an experimental contact hypersensitivity response by at least 40% at 24 hours after specific antigen challenge, or as evaluated in vivo in humans by the agent's ability to inhibit contact hypersensitivity responses to patch test allergens in patients hypersensitive to a given allergen, using procedures generally accepted by those of skill in the art, and wherein the derivative has the formula: 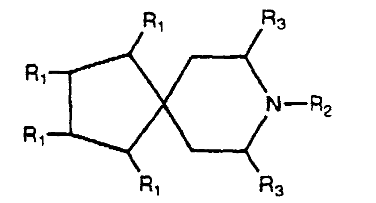 wherein:
- R₁ =: H; halo (chloro, bromo, fluoro, or iodo); alkyl, specifically including CH₃-, cyclohexyl, (CH₃)₂CH-, CH₃(CH₂)₃-, (CH₃)₂CHCH₂-, CH₃CH₂CH(CH₃)-, (CH₃)₃C-, and -CH₃(CH₂)ₚ; Y-CH₂(CH₂)ₙ-; oxyalkyl; or aryl, specifically including C₆H₅-, (2, 3, or 4)-(OCH₃)C₆H₄- and (2, 3, or 4)-(CH₃)C₆H₄-; 2-X-C₆H₄-, 3-X-C₆H₄-, or 4-X-C₆H₄-; oxyaryl; or alkaryl;
- R₂ =: H, C₆H₅CH(CH₂CH₃)CH₂-, C₆H₅CH(CH₃)(CH₂)₂-, C₆H₅CH₂CH(CH₃)CH₂-, C₆H₅CH₂CH₂CH(CH₃)-, C₆H₅CH(CH₃)(CH₂)₃-, (2, 3, or 4)-(alkyl)-C₆H₄CH(CH₃)(CH₂)₃-, (2, 3, or 4)-(alkyloxy)-C₆H₄CH(CH₃)(CH₂)₃, (2, 3, or 4)-X-C₆H₄-alkyl, specifically including
(2, 3, or 4)-X-C₆H₄CH(CH₂CH₃)CH₂-, (2, 3, or 4)-X-C₆H₄CH(CH₃)(CH₂)-, 4-X-C₆H₄CH(CH₃)(CH₂)₂-, and 4-X-C₆H₄-CH(CH₃)(CH₂)₃-; C₆H₅CH(OCH₃)(CH₂)₂-, 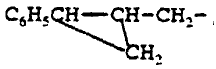 , C₆H₅CO(CH₂)₃⁻, C₆H₅CO(CH₂)₄-, (2, 3, or 4)-(alkyl)-C₆H₄CO(CH₂)₃-, (2, 3, or 4)-(alkyl-oxy)-C₆H₄CO(CH₂)₃-, (2, 3, or 4)-X-C₆H₄CO(CH₂)ₙ-, 2-thienyl-CO-(CH₂)₃-, -alkyl-piperazinyl-aryl; -alkyl-C₃₋₈cycloalkyl-aryl; -alkyl-piperazinyl-heterocycle; -alkyl-C₃₋₈cycloalkyl-heterocycle; -alkyl-C₃₋₈cycloalkyl-Ar₁; -alkyl-piperazinyl-Ar₁; -alkenyl-piperazinyl-aryl; -alkenyl-C₃₋₈cycloalkyl-aryl; -alkyl-aryl-heterocycle; -alkyl-heterocycle-aryl; -alkenyl-C₃₋₈cycloalkyl-Ar₁; -alkenyl-piperazinyl-heterocycle; -alkenyl-C₃₋₈cycloalkyl-heterocycle; -alkenyl-piperazinyl-Ar₁; 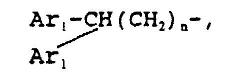 (2, 3, or 4)-X-C₆H₄C(CH₃)CH(CH₂)₂-, where the conformation about the double bond is cis or trans, (2, 3, or 4)-X-C₆H₄C(CH₃)CHCH₂-, where the conformation about the double bond is cis or trans, (2, 3, or 4)-X-C₆H₄COCH=CHCH₂-, Y-CH₂(CH₂)ₙ-, Ar₁-(CH₂)ₙ-, C₁ to C₂₀ alkyl, X-(CH₂)ₙCO-, or X-(CH₂)ₙ-;
- R₃ =: =O, =NH, =S, chloro, bromo, iodo, fluoro, alkyl, or aryl;
- n =: 1 to 6;
- p =: 1 to 20;
- X =: is independently F, Cl, Br, I, OCH₃, SO₃⁻, NH₂, H, -OH, -COOH, -COOR, -SO₃H, -CN, -NHSO₃H, -NO₂, or -SO₂NH₂;
- Y =: H, F, Cl, Br, I, -SO₃, -PO₄⁼, -OH, -SH, -SCH₃, -CH₃SO₂⁻, -NH₂, or -CO₂⁻; and
- Ar₁ =: independently, aryl, (2, 3, or 4-X-C₆H₄-), (2, 3, or 4)-(CH₂X)C₆H₄-, (2, 3, or 4)-(CX₃)C₆H₄-, (2, 3, or 4)-(CHX₂)C₆H₄-, 2-thienyl, or (2, 3, or 4)-X-C₆H₄CH₂-;
or its pharmaceutically acceptable salt, including any quaternary salt known to those in the art, and specifically including the quaternary ammonium salt of the formula -NR⁺Z⁻, wherein R is alkyl (and in particular methyl or ethyl) or benzyl, and Z is a counteranion, including chloride, bromide, iodide, -O-alkyl, toluenesulfonate, methylsulfonate, sulfonate, sulfate, phosphate, or carboxylate (such as benzoate, succinate, acetate, glycolate, propionate, maleate, malate, citrate, tartrate, ascorbate, benzoate, cinnamoate, mandeloate, benzyloate, and diphenylacetate).

Methods of synthesis of buspirone or its derivatives are disclosed in, or can be easily adapted by one of ordinary skill in organic synthesis from procedures disclosed in Wu, et al., J. Med. Chem. **15**, 477 (1972), Ger. Patent No. 2,057,845, and U.S. Patent No. 3,717,634. See also J. Clin. Psychiat. **43**, 1-116 (1982).

As demonstrated in Example 1, the parent buspirone has significant immunosuppressive activity. The potential utility of any one of the above-described buspirone derivatives to act as an immunosuppressant can be conveniently determined by synthesizing the compound and testing it in the biological assay described in Example 1.

The active compounds described herein exhibit an immunosuppressive effect when provided topically or systemically. The derivative is considered an immunosuppressant if it suppresses the ear swelling associated with an experimental contact hypersensitivity response by at least 40% at 24 hours after specific antigen challenge. Alternatively, the agent can be evaluated in vivo in humans by assessing the agent's ability to inhibit contact hypersensitivity responses to patch test allergens in patients hypersensitive to a given allergen, using procedures generally accepted by those of skill in the art, or by evaluation in an animal model, for example, of allograft rejection, experimental allergic encephalomyelitis, lupus erythematosus, Freund's adjuvant arthritis and/or graft versus host disease.

For systemic administration, derivatives of buspirone which are particularly useful are those that have an immunosuppressive effect but which do not exhibit a significant neuroleptic effect, Buspirone derivatives without significant neuroleptic effect can be identified by their ability to bind to serotonin or dopamine receptors, or by assessing their lack of ability to act as a tranquilizer or neuroleptic in mammals, for example, by demonstrating that they are no different than placebo, for example, in the hot plate test of Eddy, et al., J. Pharmacol. 107:385 (1953) and 110:135 (1954).

The chemically unrelated serotonin receptor antagonists, trazadone and mianserin, and the dopamine receptor antagonist, haloperidol, are not effective in suppressing contact hypersensitivity. On this basis, it appears that the mechanism of action of buspirone and buspirone derivatives in suppressing the immune response is independent of their serotonin or dopamine receptor blocking properties. Therefore, buspirone derivatives with immunosuppressive effects yet without neuroleptic effects can be provided by the method of selection disclosed generally herein.

### II. Complexation or Modification of the Buspirone Nucleus to Prevent Significant Neuroleptic Effect

As discussed above, immunosuppressive compounds with a buspirone nucleus that have a neuroleptic effect can be complexed or modified to eliminate that effect, by one or more of the following processes.

### A. Decreasing the Lipophilicity, or Increasing the Hydrophilicity of the Compound

Compounds with a buspirone nucleus that exhibit an immunosuppressive effect yet also exhibit a neuroleptic effect can be modified to minimize the neuroleptic effect by decreasing the lipophilicity (equivalent to increasing the hydrophilicity) of the molecule. This can be done by adding one or more charged side chain(s) onto the molecule or by altering the existing side chain to make it more polar. The hydrophilicity of buspirone derivatives will in general increase when charged substituents are added.

### B. Increasing the Size of the Molecule

Another technique for reducing the central nervous system (CNS) effects of compounds that contain a buspirone nucleus is to increase the size of the molecule via a covalent linkage to a large moiety (e.g., albumin or polyethylene glycol), using standard techniques of organic synthesis or by choosing a buspirone derivative with large substituents.

### C. Complexing the Buspirone Nucleus with a Cyclic Molecule

A third method for reducing the central nervous system (CNS) effects of a compound that contains a buspirone nucleus includes forming a non-covalent complex of the compound with a cyclic molecule such as a cycloamylose (e.g., a cyclodextrin such as β-cyclodextrin), which has a spatial arrangement of hydroxyl groups whereby the outer surface of the ring formed by the cycloamylose is hydrophilic and the inner surface is lipophilic.

When utilized in aqueous solution, this structure permits molecules (or parts thereof), termed "guest molecules", which are less polar than water and which are of suitable dimensions, to be incorporated into the lipophilic inner cavity, such that the cycloamylose/guest molecule complex presents to the blood-brain barrier as a relatively large and polar compound which is unable to penetrate the barrier. Such complexes may be prepared by any method known to the art, including those described in U.S. Patent No. 4,555,504, which discloses β-cyclodextrin complexed with digoxin.

The central nervous system side effects of a buspirone derivative can be estimated using molecular modeling and/or pharmacophore analysis. The dopamine and serotonin receptors are well characterized and strategies for estimating binding of drugs to these receptors are well established. For example, Schmidt, et al., Molecular Pharmacology 38:511-516 (1990), describe an algorithm for estimating the binding affinity of drugs to the 5-HT receptor. Also, a composite pharmacophore analysis and chemical database screening strategy is described by Sleight, et al, Naunyn-Schmiedebergs Arch. Pharmacol. 343:109-116 (1991), and Schmidt, A.W. and Peroutka, S.J., Mol. Pharmacol. 36(4):505-511 (1989).

### D. Administration as a Quaternary Salt

Buspirone or its above-defined derivative can be administered in the form of a pharmaceutically acceptable quaternary salt. Quaternary salts are typically less lipophilic than the corresponding unquaternized compound, and therefore have a decreased effect on the central nervous system. Nonlimiting examples of quaternary salts that can be used include salts prepared from methyl chloride, methyl bromide, methyl iodide, methyl sulfate, methyl benzene-sulfonate, methyl p-toluenesulfonate, ethyl chloride, ethyl bromide, ethyl iodide, n-propyl chloride, n-propyl bromide, n-butyl bromide, isobutyl bromide, sec-butyl bromide, n-amyl bromide, n-hexyl chloride, benzyl chloride, benzyl bromide, and ethyl sulfate. Other nonlimiting examples of quaternary salts specifically include the quaternary ammonium salt of the formula -NR₃⁺Z⁻, wherein R is alkyl or benzyl, and Z is a counteranion, including chloride, bromide, iodide, -O-alkyl, toluenesulfonate, methylsulfonate, sulfonate, phosphate, or carboxylate (such as benzoate, succinate, acetate, glycolate, succinate, maleate, malate, citrate, tartrate, ascorbate, benzoate, cinnamoate, mandeloate, benzyloate, and diphenylacetate).

The methyl ammonium tosylate salt of buspirone has been found to be toxic to mice at elevated dosage levels (above 10 mg/kg). Therefore, this quaternary salt of buspirone or its derivatives should be administered as the lowest dosage that achieves a desired effect.

### II. Therapeutic Compositions

Buspirone or its derivative can be included in a pharmaceutically acceptable carrier or diluent in an amount sufficient to deliver to a patient a therapeutic amount of compound in vivo in the absence of serious toxic effects for any of the above described disorders.

The concentration of active compound in the drug composition will depend on absorption, inactivation, and excretion rates of the drug as well as other factors known to those of skill in the art. It is to be noted that dosage values will also vary with the severity of the condition to be alleviated. It is to be further understood that for any particular subject, specific dosage regimens should be adjusted over time according to the individual need and the professional judgment of the person administering or supervising the administration of the compositions, and that the dosage ranges set forth herein are exemplary only and are not intended to limit the scope or practice of the claimed composition. The active ingredient may be administered once, or may be divided into a number of smaller doses to be administered at varying intervals of time.

Buspirone or its derivative can be mixed with other active materials which do not impair the desired action, or with materials that supplement the desired action, such as antibiotics, antifungals, anti-inflammatories, antivirals, or other immunosuppressive agents.

Buspirone or its derivatives can be provided in the form of pharmaceutically-acceptable salts. As used herein, the term "pharmaceutically-acceptable salts or complexes" refers to salts or complexes that retain the desired biological activity of the parent compound and exhibit minimal, if any, undesired toxicological effects. Examples of such salts are (a) acid addition salts formed with inorganic acids (for example, hydrochloric acid, hydrobromic acid, sulfuric acid, phosphoric acid, nitric acid, and the like), and salts formed with organic acids such as acetic acid, oxalic acid, tartaric acid, succinic acid, malic acid, ascorbic acid, benzoic acid, tannic acid, pamoic acid, alginic acid, polyglutamic acid, naphthalenesulfonic acids, naphthalenedisulfonic acids, and polygalacturonic acid; (b) base addition salts formed with polyvalent metal cations such as zinc, calcium, bismuth, barium, magnesium, aluminum, copper, cobalt, nickel, cadmium, and the like, or with an organic cation formed from N,N-dibenzylethylene-diamine or ethylenediamine; or (c) combinations of (a) and (b); e.g., a zinc tannate salt or the like.

Buspirone or its derivatives can be modified in order to enhance their usefulness as pharmaceutical compositions. For example, it is well know in the art that various modifications of the active molecule, such as alteration of charge, can affect water and lipid solubility and thus alter the potential for percutaneous absorption. The vehicle, or carrier, can also be modified to enhance cutaneous absorption, enhance the reservoir effect, and minimize potential irritancy or neuropharmacological effects of the composition. See, in general, Arndt, K.A., P.V. Mendenhall, "The Pharmacology of Topical Therapy", Dermatology in General Medicine, 1987; T.B. Fitzpatrick, A.Z. Eisen, K. Wolff, I.M. Freedberg and K.F. Austen, eds., 3d ed., McGraw Hill, Inc., New York, pp. 2532-2540.

Compounds that are useful are typically those that have a therapeutic index of at least 2, and preferably 5 or 10 or greater, wherein therapeutic index is defined as EC₅₀/IC₅₀.

### A. Topical Administration

Mammals, and specifically humans, suffering from pathological cutaneous, ocular, or mucosal immune responses can be treated by topical administration to the patient of an effective amount of buspirone or its derivative or its salt, optionally in combination with a pharmaceutically acceptable carrier or diluent.

The active compound is administered topically in an effective dosage range to cause immunosuppression of the target pathological immune response. The active compound is included in the pharmaceutically acceptable topical carrier or diluent in an amount sufficient to deliver to a patient a therapeutic amount of the buspirone derivative locally in the absence of serious toxic effects. In general, local immunosuppression can be achieved by topically administering lower doses of buspirone derivatives than would be required if the agents were administered systemically. Typical dosages for topical application for all of the above-identified conditions are those ranging from 0.001 to 100% by weight of the active compound.

Solutions or suspensions for topical application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The pH can be adjusted with acids or bases, such as hydrochloric acid or sodium hydroxide.

Suitable vehicles or carriers for topical application can be prepared by conventional techniques, such as lotions, suspensions, ointments, creams, gels, tinctures, sprays, powders, pastes, slow-release transdermal patches, suppositories for application to rectal, vaginal, nasal or oral mucosa mouth washes, swish and/or spit solutions. In addition to the other materials listed above for systemic administration, thickening agents, emollients, and stabilizers can be used to prepare topical compositions. Examples of thickening agents include petrolatum, beeswax, xanthan gum, or polyethylene, humectants such as sorbitol, emollients such as mineral oil, lanolin and its derivatives, or squalene. A number of solutions and ointments are commercially available, especially for ophthalmic applications.

Thickening agents, emollients, and stabilizers can be used to prepare topical compositions. Examples of thickening agents include petrolatum, beeswax, xanthan gum, or polyethylene glycol, humectants such as sorbitol, emollients such as mineral oil, lanolin and its derivatives, or squalene. A number of solutions and ointments are commercially available, especially for ophthalmic and dermatologic applications.

Natural or artificial flavorings or sweeteners can be added to enhance the taste of topical preparations applied for local effect to mucosal surfaces. Inert dyes or colors can be added, particularly in the case of preparations designed for application to oral mucosal surfaces.

Buspirone or its derivative can be applied in a time release formulation via transdermal patches or by slow release polymers. The active compounds can be prepared with carriers that will protect the compound against rapid release, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Many methods for the preparation of such formulations are patented or generally known to those skilled in the art.

### B. Systemic Administration

Mammals, and specifically humans, suffering from pathogenic immune responses can also be treated by the systemic administration to the patient of an effective amount of the buspirone derivative or its salt optionally in combination with a pharmaceutically acceptable carrier or diluent for systemic delivery.

The buspirone derivative can be administered, for example, subcutaneously, intravenously, intraperitoneally, intramuscularly, parenterally, orally, submucosally, by inhalation, or transdermally via a slow release patch, in an effective dosage range to cause systemic immunosuppression. Typical systemic dosages for all of the above-identified conditions are those ranging from 20 mg/kg to 0.0001 mg/kg per day as a single daily dose or divided daily doses. The effective dosage of the parent compound, buspirone, for systemic immunosuppression is believed to be higher than the effective dosage of buspirone for inducing a neuroleptic effect.

The buspirone derivative is administered systemically for a sufficient time period to alleviate the undesired symptoms and the clinical signs associated with the condition being treated.

A preferred mode of systemic administration of the active compound is oral. Oral compositions will generally include an inert diluent or an edible carrier. They may be enclosed in gelatin capsules or compressed into tablets. For the purpose of oral therapeutic administration, the active compound can be incorporated with excipients and used in the form of tablets, troches, or capsules. Pharmaceutically compatible binding agents, and/or adjuvant materials can be included as part of the composition.

The tablets, pills, capsules, troches and the like can contain any of the following ingredients, or compounds of a similar nature: a binder such as microcrystalline cellulose, gum tragacanth or gelatin; an excipient such as starch or lactose, a disintegrating agent such as alginic acid, Primogel, or corn starch; a lubricant such as magnesium stearate or Sterotes; a glidant such as colloidal silicon dioxide; a sweetening agent such as sucrose or saccharin; or a flavoring agent such as peppermint, methyl salicylate, or orange flavoring.

When the dosage unit form is a capsule, it can contain, in addition to material of the above type, a liquid carrier such as a fatty oil. In addition, dosage unit forms can contain various other materials which modify the physical form of the dosage unit, for example, coatings of sugar, shellac, or other enteric agents.

The buspirone derivative or its salts can be administered as a component of an elixir, suspension, syrup, wafer, chewing gum or the like. A syrup may contain, in addition to the active compounds, sucrose as a sweetening agent and certain preservatives, dyes and colorings and flavors.

Solutions or suspensions used for parenteral, intradermal, or subcutaneous, application can include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. pH can be adjusted with acids or bases, such as hydrochloric acid or sodium hydroxide. The parenteral preparation can be enclosed in ampoules, disposable syringes or multiple dose vials made of glass or plastic.

If administered intravenously, preferred carriers are bacteriostatic water, physiological saline, Cremophor EL™ (BASF, Parsippany, NJ) or phosphate buffered saline (PBS).

In one embodiment, the active compounds are prepared with carriers that will protect the compound against rapid elimination from the body on systemic delivery, such as a controlled release formulation, including implants and microencapsulated delivery systems. Biodegradable, biocompatible polymers can be used, such as ethylene vinyl acetate, polyanhydrides, polyglycolic acid, collagen, polyorthoesters, and polylactic acid. Methods for preparation of such formulations will be apparent to those skilled in the art.

Liposomal suspensions (including liposomes targeted to infected cells with monoclonal antibodies to specific antigens) can also be used as pharmaceutically acceptable carriers. These may be prepared according to methods known to those skilled in the art, for example, as described in U.S. Patent No. 4,522,811. For example, liposome formulations may be prepared by dissolving appropriate lipid(s) (such as stearoyl phosphatidyl ethanolamine, stearoyl phosphatidyl choline, arachadoyl phosphatidyl choline, and cholesterol) in an organic solvent that is then evaporated, leaving behind a thin film of dried lipid on the surface of the container. An aqueous solution of the buspirone derivative is then introduced into the container. The container is then swirled by hand to free lipid material from the sides of the container and to disperse lipid aggregates, thereby forming the liposomal suspension.

### III. Immunosuppressant Activity of Buspirone Derivatives

Buspirone and buspirone derivatives are capable of acting systemically or topically to suppress the immune response in humans and other mammals. As such, the compounds, or therapeutic compositions thereof, are useful for the treatment of a myriad of immunological disorders or other pathological conditions associated with an immune response. Examples of such disorders include cutaneous contact hypersensitivity, host rejection of foreign organ or tissue transplants; graft-vs-host disease in which donor immunological cells present in the graft attack host tissues in the recipient of the graft; diseases with proven or possible autoimmune components, such as rheumatoid arthritis and juvenile rheumatoid arthritis, aphthous ulcer, lichen planus, psoriatic arthritis, psoriasis, excema, conjunctivitis, Sjogren's Syndrome, including keratoconjunctivitis sicca secondary to Sjogren's Syndrome, iritis, alopecia areata, cutaneous lupus erythematosus, scleroderma, vaginitis, proctitis, drug eruptions, leprosy reversal reactions, erythema nodosum leprosum, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener's granulomatosis, chronic active hepatitis, myasthenia gravis, Stevens-Johnson syndrome, idiopathic sprue, Crohn's disease, ulcerative colitis, Graves ophthalmopathy, sarcoidosis, primary biliary cirrhosis, primary juvenile diabetes, uveitis posterior, and interstitial lung fibrosis; allergic asthma; allergic responses due to arthropod bite reactions, asthma, allergic asthma, and inappropriate allergic responses to other environmental stimuli such as atopic dermatitis and hypersensitivity to pollen, insect stings and certain foods. These compounds can also be useful in reducing the infiltration of skin by malignant leukocytes in diseases such as mycosis fungoides.

Buspirone and its derivatives can also be used to increase tear production in a patient suffering from deficient tears in the eye due to an autoimmune dysfunction of the lacrimal glands, such as immune mediated keratoconjunctivitis (KCS, or dry eye). Canine KCS is a common, chronic progressive, and potentially blinding disease. A continuum of corneal and conjunctival lesions ensues from the dry eye state. Buspirone or its active derivatives can be provided as an ophthalmic drop or ophthalmic ointment to humans or other mammals, including dogs and cats, in an effective amount in a suitable vehicle. This topical ophthalmic treatment can also serve to correct corneal and conjunctival disorders exacerbated by tear deficiency and KCS, such as corneal scarring, corneal ulceration, filamentary keratitis, mucopurulent discharge, and vascularization of the cornea. Buspirone and its derivatives can also be used to decrease immune responses which contribute to granulation and neovascularation in the cornea.

The ability of buspirone to influence the tissue swelling associated with contact hypersensitivity reactions in mice was evaluated as described in detail in Example 1. Buspirone HCl was used for the procedure in Example 1 as a model of an active immunosuppressant. Buspirone derivatives can be measured against this model, and are considered active if they suppress the swelling response by at least 40% 24 hours after specific antigen challenge.

When applied topically, preparations of buspirone significantly suppressed the tissue swelling associated with the elicitation phase of contact hypersensitivity to oxazolone. However, mice treated topically with buspirone HCl, unlike those treated systemically, exhibited no drowsiness or other evidence of central nervous system effects.

Buspirone expresses both serotonin and dopamine receptor antagonist activity. However, unlike buspirone, it was discovered that the chemically unrelated serotonin antagonists, trazadone and mianserin, and the dopamine receptor antagonist, haloperidol, were not effective in suppressing contact hypersensitivity. On the basis of this, it appears that the mechanism of action of buspirone on the immune response is independent of its serotonin or dopamine receptor blocking properties, and therefore, buspirone derivatives with immunosuppressive effect yet without neuroleptic effect can be provided by the method of selection disclosed generally herein.

### Example 1: Inhibition of Induced Contact Hypersensitivity.

Six-to-8-week-old female C57BL/6J or BALB/c mice were obtained from the Jackson Laboratory, Bar Harbor, Maine or from Charles River Laboratories, Kingston Facility, Stoneridge, NY, respectively.

Buspirone HCl, mianserin, trazadone, haloperidol and oxazolone were purchased from the Sigma Chemical Co. (St. Louis, NO).

Oxazolone-Induced Contact Hypersensitivity - Sensitization and challenge for contact hypersensitivity were performed as follows. The abdomens of the mice were shaved with electric clippers, 50 µl of a 4% (w/w) solution of oxazolone in 4:1 (v:v) acetone:olive oil were applied to the shaved abdomen, and 5 µl of the same solution were applied to each hind footpad. Five to eight days later, the mice were challenged for contact hypersensitivity by applying 10 µl of a 0.5% (w:w) solution of oxazolone in 4:1 (v:v) acetone:olive oil to both the inner and outer surface of the right ear of each mouse (in the case of mice treated systemically with buspirone HCl) or to both ears (in the case of mice treated topically with buspirone HCl) - except in the case where sensitization phase suppression is studied, as in Figures 9 and 10.

Systemic Buspirone HCl Treatment - One hour after the application of oxazolone for elicitation of contact hypersensitivity, mice were treated subcutaneously with buspirone HCl 500 or 50 mg/kg body weight) in 0.1 mL of carrier (Cremophor EL, BASF, Parsippany, NJ), or with 0.1 mL of carrier alone. In a separate experiment, mice were treated in a similar fashion with 50 mg/kg body weight of trazadone, mianserin, haloperidol, or buspirone HCl in 1 mL olive oil or with olive oil alone.

Topical Buspirone HCl Treatment For these experiments, both ears of each mouse were challenged for elicitation of contact hypersensitivity by the application of oxazolone (as appropriate) to both surfaces of both ears. Two hours before, or twenty-four hours after application of hapten, the right ears of some mice were treated with buspirone HCl in vehicle, applied epicutaneously to both surfaces. The right ears of control mice were similarly treated, but with vehicle alone. In the case of experiments designed to assess topical effects on the sensitization phase, only the right ear is challenged. (See Figures 9 and 10)

Evaluation of Ear Swelling Response - Immediately before and 24 or 48 hours after application of oxazolone, ear thicknesses were determined with an engineer's micrometer. The increment (delta) in ear thickness (ear swelling) was calculated as the 24- or 48-hour value minus the baseline (pre-challenge) value and expressed in units of 10⁻⁴ inches. Mice were killed by cervical dislocation after the measurement of 24-hour ear thickness was obtained, and the ears were processed for histologic examination.

Quantification of Leukocyte Infiltration - Both ears of each mouse were fixed in 4.0% buffered formalin and then processed routinely and embedded in paraffin for preparation of 6-7 µm-thick hematoxylin and eosin-stained sections. All of the sections were coded and examined with an ocular grid at 400x under light microscopy by an observer unaware of the identity of the individual slides. The number of leukocytes/mm² of dermis was calculated by counting all of the leukocyte cells in an area of at least 0.14 mm² of dermis.

Statistical Analysis - Differences between groups were assessed by the 2-tailed Student's *t* test (paired for comparisons of left and right ears in the same mice, unpaired for comparisons between different groups of mice).

Effect of Topical Buspirone HCl on Expression of Contact Hypersensitivity - Figures 1 and 2 illustrate the effect of topical application of 100 mg/mL of buspirone HCl (Group B) or carrier alone (Group A) on expression of contact hypersensitivity. As indicated, topical administration of buspirone HCl at 100 mg/mL significantly decreased ear swelling (Figure 1) and aggregation of leukocytes (Figure 2). Importantly, while topical application of buspirone HCl was extremely effective in diminishing both the tissue swelling and the leukocyte infiltration associated with contact hypersensitivity reactions, these effects were observed in the absence of detectable alterations in the behavior of the mice. The mice treated topically with buspirone HCl appeared active and retained apparently normal interest in food and water.

Effect of Systemic Buspirone Versus Other Serotonin or Dopamine Receptor Antagonists - In these experiments, systemic buspirone was compared to the serotonin receptor antagonists, trazadone or mianserin, and to the dopamine receptor antagonist, haloperidol, for their ability to inhibit cutaneous contact hypersensitivity. At a dose of 50 mg/kg, only buspirone HCl significantly reduced cutaneous contact hypersensitivity (Figures 3 and 4).

Effect of Topically Administered Buspirone on Tissue Swelling Associated with Oxazolone-Induced Contact Hypersensitivity Reactions/Effect of Time of Administration of Topical Buspirone. Oxazolone was applied to both ears of all mice at different times either pre- or post-buspirone treatment, and the change in ear thickness was measured at a specified interval thereafter. a. Two hours before oxazolone challenge, 100 mg/mL buspirone HCl in Vehicle-N was applied to both surfaces of the right ears of some mice, whereas vehicle alone was applied to both surfaces of the ears of the control (0% buspirone) animals. The ears were measured 24 hours after oxazolone challenge (Figure 5a). Local pre-challenge treatment of the right ear with buspirone HCl significantly suppressed tissue swelling in the treated ear (***p*<0.01 vs contralateral oxazolone treated ears or vs right ears of vehicle treated group). Treatment of the right ear with 100 mg/mL buspirone HCl had no significant effect on the magnitude of swelling in the contralateral oxazolone treated ear. b. In a separate experiment, twenty-four hours after oxazolone challenge, 100 mg/mL buspirone HCl in Vehicle-N was applied to both surfaces of the right ears of some mice, whereas vehicle alone was applied to both surfaces of the ears of control (0% buspirone) mice. The change in ear thickness was determined 24 hours after treatment with buspirone, i.e. at 48 hours after challenge with oxazolone (Figure 5b). Treatment with buspirone HCl significantly diminished contact hypersensitivity reactions in the right ears of the treated animals (*=*p*<0.01 when compared to the right ears in the control mice, and *p*<0.05 when compared to the contralateral ears of the same mice). The reactions in the left ears of the mice treated on the right ears with buspirone HCl were not reduced wnen compared to reactions in the left ears of the vehicle-treated mice. When the ears of the same mice shown in Figures 5a and 5b were examined histologically (Figures 6a and 6b), buspirone HCl treatment was shown to diminish significantly the leukocyte infiltration associated with the contact hypersensitivity response (**=p<0.01 when compared to the right ears in the vehicle-tested mice).

Effect of Systemic Treatment with Buspirone on Expression of Contact Hypersensitivity - The subcutaneous administration of buspirone HCl at dosages of 5.00 or 50 mg/kg, 1 hour after challenge markedly diminished the tissue swelling which developed in association with the contact hypersensitivity response (Figure 7). The leukocyte infiltration associated with the response in mice treated with 500 or 50 mg/kg buspirone HCl was also diminished compared to responses in mice not treated with the drug (Figure 8). However, at these dosages, buspirone HCl also produced other remarkable systemic effects. The mice rapidly became lethargic after administration of the drug, and, by 23 hours after buspirone HCl injection, the mice exhibited profound depression of central nervous system function (these effects were more pronounced in the high dosage group). They appeared to be in a deep sleep, neither ate nor drank, and responded weakly or not at all to touch. They did, however, exhibit responsiveness to pinch, in both dosages.

### Example 2: Comparison of Immunosuppressant versus Anti-inflammatory activity.

Mice were sensitized to oxazolone as described in Example 1. Three days later, slow release indomethacin pellets (0.05 mg, 3 week release) were implanted subcutaneously under light ether anesthesia. The dose of indomethacin delivered by these pellets has been previously shown to completely block prostaglandin synthesis in mice, by Jun, D.D., et al., J. Invest. Dermatol. 90:311 (1988).

Three days later, mice were challenged for contact hypersensitivity as in Example 1. When the hypersensitivity response was assessed 24 hours later, indomethacin was shown to have no significant effect on the response. These figures (11 and 12) show that a classic anti-inflammatory agent, indomethacin, does not appear to suppress the edema associated with the immunologically specific oxazolone induced contact hypersensitivity response and compared to buspirone HCl, only weakly suppresses the leukocyte infiltration associated with the response.

### Example 3: Evaluation of Serotonin Receptor Binding Activity or Dopamine Receptor Binding Activity of Buspirone Derivatives.

Buspirone derivatives which lack serotonin receptor binding or dopamine receptor binding activity can be identified as follows. A radiolabeled ligand known to bind serotonin and/or dopamine receptors can be bound to an appropriate substrate expressing one or both of these receptors. For example, radiolabeled quipazine which is available commercially can be used as the ligand. The buspirone derivative to be tested is then incubated with the radiolabeled quipazine ligand combination. Displacement of radiolabeled ligand is positive evidence that the buspirone derivative being tested can bind serotonin and/or dopamine receptors. The amount of radiolabeled ligand which is displaced is determined by an appropriate standard curve which can also provide information concerning binding affinities. The displaced radiolabeled ligand can be quantitated using a standard scintillation counter.

A detailed description of how to perform the binding studies using ³H-quipazine and the example follows:

Binding studies using ³H-quipazine are described in detail by Milburn, C.M. and Peroutka, S.J., J. Neurochem. 52:1787-1792 (1989). Briefly, rat cortices are homogenized in 20 volumes of 50 mM Tris HCl buffer pH 7.7 at 25°C and centrifuged at 49,000 x g for 10 min. The pellet is resuspended in fresh buffer and incubated at 37°C for 10 min-After the final centrifugation, the pellet is resuspended in 80 volumes of Krebs-HEPES buffer (25 mM HEPES, 118 mM NaCl, 5 mM KCl, 2.5 mM CaCl₂, and 1.2 mM MgCl₃ pH adjusted to 7.4). Tissue (10 mg of original wet weight) is added to assay tubes containing 0.8 nM [³H]quipazine and displacing drug or buffer in a final volume of 1 mL. Non-specific binding is defined using 1 micromole zacopride. After a 30 min incubation at room temperature, the tissue is rapidly filtered under vacuum through No. 32 glass fiber filters and rinsed twice with 5 mL of 50 mM Tris-HCl buffer pH 7.7. Radioactivity is quantified by liquid scintillation counting. All experiments are performed three to six times, each in triplicate. This same approach can be used with other radiolabeled ligands such as zacopride, granisetron, haloperidol, mianserin, ketanserin, 5-HT, dopamine, droperidol, or ritanserin.

Buspirone derivatives which have binding affinities for dopamine and/or serotonin receptors of one/tenth or less than native buspirone are considered to be potentially useful as systemic immunosuppressants if they are at least 50% as active as native buspirone on a weight basis in suppressing immunologically specific responses such as contact hypersensitivity.

## Claims

1. Use of a compound selected from the group consisting of buspirone and a buspirone derivative of the formula 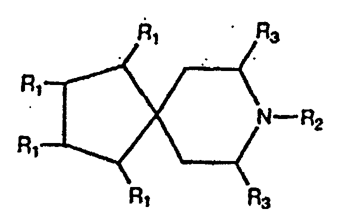 wherein:
R₁ = H; halo (chloro, bromo, fluoro, or iodo); alkyl, specifically including CH₃-, cyclohexyl, (CH₃)₂CH-, CH₃(CH₂)₃-, (CH₃)₂CHCH₂-, CH₃CH₂CH(CH₃)-, (CH₃)₃C-, and -CH₃(CH₂)ₚ; Y-CH₂(CH₂)ₐ-; oxyalkyl; C₆H₅-, aryl, (2, 3, or 4)-(OCH₃)C₆H₄- and (2, 3, or 4)-(CH₃)C₆H₄-; 2-X-C₆H₄-, 3-X-C₆H₄-, or 4-X-C₆H₄-; oxyaryl; or alkaryl;
R₂ = H, C₆H₅CH(CH₂CH₃)CH₂-, C₆H₅CH(CH₃)(CH₂)₂-, C₆H₅CH₃CH(CH₃)CH₂-, C₆H₅CH₂CH₃CH(CH₃)-, C₆H₅CH(CH₃)(CH₂)₃-, (2, 3, or 4)-(alkyl)-C₆H₄CH(CH₃)(CH₂)₃-, (2, 3, or 4)-(alkyloxy)-C₆H₄CH(CH₃)(CH₂)₃, (2, 3, or 4)-X-C₆H₄-alkyl, (2, 3, or 4)-X-C₆H₄CH(CH₂CH₃)CH₂-, (2, 3, or 4)-X-C₆H₄CH(CH₃)(CH₂)- 4-X-C₆H₄CH(CH₃)(CH₂)₂-, and 4-X-C₆H₄-CH(CH₃)(CH₂)₃-; C₆H₅CH(OCH₃)(CH₂)₂-, 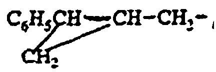 , C₆H₅CO(CH₂)₃-, C₆H₅CO(CH₂)₄-, (2, 3, or 4)-(alkyl)-C₆H₄CO(CH₂)₃-, (2, 3, or 4)-(alkyl-oxy)-C₆H₄CO(CH₂)₃-, (2, 3, or 4)-X-C₆H₄CO(CH₂)ₙ-, 2-thienyl-CO-(CH₂)₃-, -alkyl-piperazinyl-aryl; -alkyl-C₃₋₈cycloalkyl-aryl; -alkyl-piperazinyl-heterocycle; -alkyl-C₃₋₈cycloalkyl-heterocycle; -alkyl-C₃₋₈cycloalkyl-Ar₁; -alkyl-piperazinyl-Ar₁; -alkenyl-piperazinyl-aryl; -alkenyl-C₃₋₈cycloalkyl-aryl; -alkyl-aryl-heterocycle; -alkyl-heterocycle-aryl; -alkenyl-C₃₋₈cycloalkyl-Ar₁; -alkenyl-piperazinyl-heterocycle; -alkenyl-C₃₋₈-cycloalkyl-heterocycle; -alkenyl-piperazinyl-Ar₁; 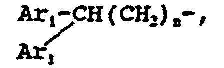 , (2, 3, or 4)-X-C₆H₄C(CH₃)CH(CH₂)₂-, where the conformation about the double bond is cis or trans, (2, 3, or 4)-X-C₆H₄C(CH₃)CHCH₂-, where the conformation about the double bond is cis or trans, (2, 3, or 4)-X-C₆H₄COCH=CHCH₂-, Y-CH₂(CH₂)ₙ-, Ar₁-(CH₂)ₙ-, C₁ to C₂₀ alkyl, X-(CH₂)ₙ-CO-, or X-(CH₂)ₙ-;
R₃ = =O, =NH, =S, chloro, bromo, iodo, fluoro, alkyl, or aryl;
n = 1 to 6;
p = 1 to 20;
X = is independently F, Cl, Br, I, OCH₃, SO₃⁻, NH₂, H, -OH, -COOH, -COOR, -SO₃H, -CN, -NHSO₃H, -NO₂, or -SO₂NH₂;
Y = H, F, Cl, Br, I, -SO₃, -PO₄⁼, -OH, -SH, -SCH₃, -CH₃SO₂⁻, -NH₂, or -CO₂⁻; and
Ar₁ = independently, aryl, (2, 3, or 4-X-C₆H₄-), (2, 3, or 4)-(CH₂X)C₆H₄-, (2, 3, or 4)-(CX₃)C₆H₄-, (2, 3, or 4)-(CHX₂)C₆H₄-, 2-thienyl, or (2, 3, or 4)-X-C₆H₄CH₂-
or its pharmaceutically acceptable salt, including a quaternary salt, in the manufacture of a medicament for treating a cutaneous, ocular, or mucosal pathology associated with an immune response in a human or other mammal wherein the medicament is for topical application wherein
alkyl refers to a saturated straight, branched, or cyclic hydrocarbon of C₁ to C₂₀, including methyl, ethyl, propyl, isopropyl, butyl, isobutyl, t-butyl, pentyl, cyclopentyl, isopentyl, neopentyl, hexyl, isohexyl, cyclohexyl, 3-methylpentyl, 2,2-dimethylbutyl, and 2,3-dimethylbutyl, and wherein
aryl refers to phenyl or substituted phenyl, wherein the substituent is independently halo, alkyl, or oxy(alkyl) (for example, methyoxy or ethoxy) and wherein the aryl can have up to three substituents, and wherein aralkyl refers to an aryl group with an alkyl substituent.

2. The use of claim 1, wherein the alkyl group is selected from the group consisting of CH₃-, cyclohexyl, (CH₃)₂CH-, CH₃(CH₂)₃-, (CH₃)₂CHCH₂-, CH₃CH₂CH(CH₃)-, -CH₃(CH₂)ₚ and (CH₃)₃C-.

3. The use of claim 1, wherein the aryl group is selected from the group consisting of C₆H₅-, (2, 3, or 4)-(OCH₃)C₆H₄- and (2, 3, or 4)-(CH₃)C₆H₄-; 2-X-C₆H₄-, 3-X-C₆H₄-, 4-X-C₆H₄-.

4. The use of claim 1, wherein (2, 3, or 4)-X-C₆H₄-alkyl- is selected from the group consisting of (2, 3, or 4)-X-C₆H₄CH(CH₂CH₃)CH₂-, (2, 3, or 4)-X-C₆H₄CH(CH₃) (CH₂)-, (2, 3, or 4)-X-C₆H₄CH(CH₃) (CH₂)₂-, and (2, 3, or 4)-X-C₆H₄-CH(CH₃) (CH₂)₃-.

5. The use of claim 1, wherein the quaternary ammonium salt is of the formula -NR₃⁺Z⁻, wherein R is alkyl or benzyl, and Z is a counteranion selected from the group consisting of chloride, bromide, iodide, -O-alkyl, toluenesulfonate, methylsulfonate, sulfonate, phosphate, benzoate, succinate, acetate, glycolate, succinate, maleate, malate, citrate, tartrate, ascorbate, benzoate, cinnamoate, mandeloate, benzyloate, and diphenylacetate.

6. The use of claim 1 wherein the mammal is a human.

7. The use of claim 1, wherein the pathology associated with an immune response is selected from the group consisting of contact dermatitis, atopic dermatitss, Sjogren's Syndrome, including keratoconjunctivitis sicca secondary to Sjogren's Syndrome, eczematous dermatitis, drug eruptions, lichen planus, psoriasis, alopecia areata, cutaneous lupus erythematosus, scleroderma, asthma, allergic asthma, ulcerative colitis, Crohn's disease, ulcerative colitis allergic reactions secondary to arthropod bite reactions, aphthous ulcers, conjunctivitis, iritis, keratoconjunctivitis, vaginitis, and proctitis.

8. The use of claim 1, wherein the medicament is a mouthwash.

9. The use of claim 1, wherein the medicament is a swish and spit solution.

10. The use of claim 1 wherein the compound is in combination with an ophthalmic carrier and the medicament is for topical application to the eye.

11. The use of claim 1, wherein the medicament is for cutaneous application.

12. The use of claim 1, wherein the medicament is for application to mucosal membranes.

13. The use of claim 1, wherein the compound is for application in a concentration of 100 mg/ml.

14. The use of claim 1, wherein the compound is present in the medicament in a concentration between 0.001% and 100%.

15. The use of claim 1 wherein the medicament comprises a time release formulation.

16. The method of claim 1, wherein the medicament comprises a retention enema.

17. The use of claim 1, wherein the medicament is for administration in combination with a compound selected from the group consisting of antivirals, antifungals, antibiotics, anti-inflammatories, and other immunosuppressants and bronchodilators or other therapeutic agents for asthma.

18. Use of a compound as defined in any one of claims 1 to 5 without significant neuroleptic effect in the manufacture of a medicament for treating a mammal in need of immunosuppression, wherein the medicament is for systemic administration.

19. The use of claim 18 wherein the mammal is a human.

20. The use of claim 18, wherein the mammal in need of immunosuppression has a disorder selected from the group consisting of autoimmune diseases, diseases of known or unknown etiology having an immunological component, and allergies.

21. The use of claim 20, wherein the autoimmune disease is selected from a group consisting of rheumatoid arthritis, juvenile rheumatoid arthritis, psoriatic arthritis, psoriasis, leprosy reversal reactions, erythema nodosum leprosum, autoimmune uveitis, multiple sclerosis, allergic encephalomyelitis, systemic lupus erythematosis, acute necrotizing hemorrhagic encephalopathy, idiopathic bilateral progressive sensorineural hearing loss, aplastic anemia, pure red cell anemia, idiopathic thrombocytopenia, polychondritis, scleroderma, Wegener's granulomatosis, chronic active hepatitis, myastbenia gravis, atopic dermatitis, Stevens-Johnson syndrome, idiopathic sprue, Crohn's disease, ulcerative colitis, Graves ophthalmopathy, sarcoidosis, primary biliary cirrhosis, primary juvenile diabetes, uveitis posterior, and interstitial lung fibrosis.

22. The use of Claim 18 wherein buspirone or the buspirone derivative is for administration by intravenous injection.

23. The use of claim 22, wherein buspirone or the buspirone derivative is for oral administration.

24. The use of claim 18, wherein buspirone or the buspirone derivative, or its pharmaceutically acceptable salt, other than a quaternary salt, is for administration via transdennal patch.

25. The use of claim 18, wherein the dosage is between 20 mg/kg and 0.0001 mg/kg of body weight per day as a single daily dose or divided daily doses.

26. The use of claim 18, wherein buspirone or the buspirone derivative to be administered is in a time release formulation.

27. The use of claim 18, wherein buspirone or its derivative is to be administered subcutaneously.

28. The use of claim 18, wherein the patient is being, has been or will be administered a compound or compounds selected from the group consisting of antivirals, antifungals, antibiotics, anti-inflammatories, and other immunosuppressants.

29. The use of claim 18 wherein the medicament further comprises a compound or compounds selected from the group consisting of antivirals, antifungals, antibiotics, anti-inflammatories, and other immunosuppressants.

30. A pharmaceutical composition for systemic administration comprising a buspirone derivative without significant neuroleptic effect as defined in any one of claims 1 to 5.

31. The composition of claim 30 in a topical carrier in an amount by weight of between 0.001 and 100% buspirone derivative.

32. The composition of claim 30, wherein the buspirone derivative comprises buspirone complexed with a cycloamylose.

33. The composition of claim 30, wherein the buspirone derivative is in a time release formulation.

34. The composition of claim 30, wherein the buspirone derivative is in combination with a compound or compounds selected from the group consisting of antivirals, antifungals, antibiotics, anti-inflammatories, and other immunosuppressants.

35. A pharmaceutical composition for topical delivery comprising an effective amount of a buspirone derivative as defined in any one of claims 1 to 5.

36. The composition of claim 35 in a topical carrier in an amount by weight of between 0.001 and 100% buspirone derivative.

37. The use of claim 25, wherein the dosage is between 10 mg/kg and 0.0001 mg/kg of body weight per day as a single daily dose or divided daily doses.

## Patentansprüche

1. Verwendung einer Verbindung, die aus der Gruppe ausgewählt ist, die aus Buspiron und einem Buspiron-Derivat der Formel 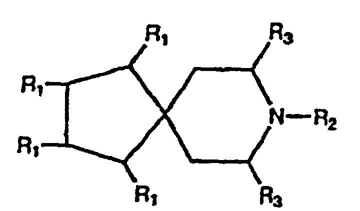 besteht, wobei:
R₁= H; ein Halogen (Chlor, Brom, Fluor oder Iod); Alkyl, insbesondere umfassend CH₃-, Cyclohexyl, (CH₃)₂CH-, CH₃(CH₂)₃-, (CH₃)₂CHCH₂-, CH₃CH₂CH(CH₃)-, (CH₃)₃C- und -CH₃(CH₂)ₚ; Y-CH₂(CH₂)ₙ-; Oxyalkyl; C₆H₅-, Aryl, (2, 3 oder 4)-(OCH₃)C₆H₄- und (2, 3 oder 4)-(CH₃)C₆H₄-; 2-X-C₆H₄-, 3-X-C₆H₄- oder 4-X-C₆H₄-; Oxyaryl; oder Alkaryl;
R₂ = H, C₆H₅CH(CH₂CH₃)CH₂-, C₆H₅CH(CH₃)(CH₂)₂-, C₆H₅CH₂CH(CH₃)CH₂-, C₆H₅CH₂CH₃CH(CH₃)-, C₆H₅CH(CH₃)(CH₂)₃-, (2, 3 oder 4)-(Alkyl)-C₆H₄CH(CH₃)(CH₂)₃-, (2, 3 oder 4)-(Alkyloxy)-C₆H₄CH(CH₃)(CH₂)₃-, (2, 3 oder 4)-X-C₆H₄-alkyl, (2, 3 oder 4)-X-C₆H₄CH(CH₂CH₃)CH₂-, (2, 3 oder 4)-X-C₆H₄CH(CH₃)(CH₂)-, 4-X-C₆H₄CH(CH₃)(CH₂)₂- und 4-X-C₆H₄-CH(CH₃)(CH₂)₃-; C₆H₅CH(OCH₃)(CH₂)₂- 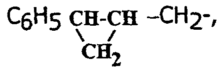 C₆H₅CO(CH₂)₃-, C₆H₅CO(CH₂)₄-, (2, 3 oder 4)-(Alkyl)-C₆H₄CO(CH₂)₃-, (2, 3 oder 4)-(Alkyloxy)-C₆H₄CO(CH₂)₃-, (2, 3 oder 4)-X-C₆H₄CO(CH₂)ₙ-, 2-Thienyl-CO-(CH₂)₃-, -Alkylpiperazinylaryl; -Alkyl-C₃₋₈-cycloalkylaryl; -Alkylpiperazinyl-Heterocyclus; -Alkyl-C₃₋₈-cycloalkyl-Heterocyclus; -Alkyl-C₃₋₈-cycloalkyl-Ar₁; -Alkylpiperazinyl-Ar₁; -Alkenylpiperazinylaryl; -Alkenyl-C₃₋₈-cycloalkylaryl; -Alkylaryl-Heterocyclus; -Alkyl-Heterocyclus-Aryl; -Alkenyl-C₃₋₈-cycloalkyl-Ar₁; -Alkenylpiperazinyl-Heterocyclus; -Alkenyl-C₃₋₈-cycloalkyl-Heterocyclus; -Alkenylpiperazinyl-Ar₁; 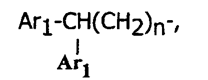 , (2, 3 oder 4)-X-C₆H₄C(CH₃)CH(CH₂)₂-, wobei die Struktur an der Doppelbindung cis oder trans ist, (2, 3 oder 4)-X-C₆H₄C(CH₃)CHCH₂-, wobei die Struktur an der Doppelbindung cis oder trans ist, (2, 3 oder 4)-X-C₆H₄COCH=CHCH₂-, Y-CH₂(CH₂)ₙ-, Ar₁-(CH₂)ₙ-, C₁-C₂₀-Alkyl, X-(CH₂)ₙCO- oder X-(CH₂)ₙ-;
R₃ = =O, =NH, =S, Chlor, Brom, Iod, Fluor, Alkyl oder Aryl;
n = 1 bis 6;
p = 1 bis 20;
X = unanbhängig F, Cl, Br, I, OCH₃, SO₃⁻, NH₂, H, -OH, -COOH, -COOR, -SO₃H, -CN, -NHSO₃H, -NO₂ oder -SO₂NH₂;
Y = H, F, Cl, Br, I, -SO₃, -PO₄⁼, -OH, -SH, -SCH₃, -CH₃SO₂⁻, -NH₂ oder -CO₂⁻; und
Ar₁ = unabhängig Aryl, (2, 3 oder 4-X-C₆H₄-), (2, 3 oder 4)-(CH₂X)C₆H₄-, (2, 3 oder 4)-(CX₃)C₆H₄-, (2, 3 oder 4)-(CHX₂)C₆H₄-, 2-Thienyl oder (2, 3 oder 4)-X-C₆H₄CH₂-
oder eines pharmazeutisch verträglichen Salzes davon, einschließlich eines quatemären Salzes, bei der Herstellung eines Medikamentes für die Behandlung einer mit einer Immunreaktion verbundenen Haut-, Augen- oder Schleimhauterkrankung beim Menschen oder bei einem anderen Säuger, wobei das Medikament für die topische Anwendung vorgesehen ist, wobei
Alkyl einen gesättigten geradkettigen, verzweigten oder cydischen Kohlenwasserstoff mit C₁ bis C₂₀, einschließlich Methyl, Ethyl, Propyl, Isopropyl, Butyl, Isobutyl, t-Butyl, Pentyl, Cyclopentyl, Isopentyl, Neopentyl, Hexyl, Isohexyl, Cyclohexyl, 3-Methylpentyl, 2,2-Dimethylbutyl und 2,3-Dimethylbutyl, bezeichnet, und wobei
Aryl eine Phenyl- oder substituierte Phenylgruppe bezeichnet, wobei der Substituent unabhängig ein Halogen, Alkyl oder Oxy(alkyl) (z.B. Methoxy oder Ethoxy) ist und
wobei Aryl bis zu drei Substituenten aufweisen kann und wobei Aralkyl eine Arylgruppe mit einem Alkylsubstituenten bezeichnet.

2. Verwendung nach Anspruch 1,
wobei die Alkylgruppe aus der Gruppe ausgewählt ist, die aus CH₃-, Cyclohexyl, (CH₃)₂CH-, CH₃(CH₂)₃-, (CH₃)₂CHCH₂-, CH₃CH₂(CH(CH₃)-, -CH₃(CH₂)p und (CH₃)₃C- besteht.

3. Verwendung nach Anspruch 1,
wobei die Arylgruppe aus der Gruppe ausgewählt ist, die aus C₆H₅-, (2, 3 oder 4)-(OCH₃)C₆H₄- und (2, 3 oder 4)-(CH₃)C₆H₄-; 2-XC₆H₄-, 3-X-C₆H₄-, 4-X-C₆H₄- besteht.

4. Verwendung nach Anspruch 1,
wobei (2, 3 oder 4)-X-C₆H₄-alkyl aus der Gruppe ausgewählt ist, die aus
(2, 3 oder 4)-X-C₆H₄CH(CH₂CH₃)CH₂-,
(2, 3 oder 4)-X-C₆H₄CH(CH₃)(CH₂)-,
(2, 3 oder 4)-X-C₆H₄CH(CH₃)(CH₂)₂- und
(2, 3 oder 4)-X-C₆H₄-CH(CH₃)(CH₂)₃- besteht.

5. Verwendung nach Anspruch 1,
wobei das quaternäre Ammoniumsalz die Formel -NR₃⁺Z⁻ hat, wobei R Alkyl oder Benzyl ist und Z ein Gegen-Anion ist, das aus der Gruppe ausgewählt ist, die aus Chlorid, Bromid, Iodid, -O-alkyl, Toluolsulfonat, Methylsulfonat, Sulfonat, Phosphat, Benzoat, Succinat, Acetat, Glycolat, Succinat, Maleat, Malat, Citrat, Tartrat, Ascorbat, Benzoat, Cinnamoat, Mandeloat, Benzyloat und Diphenylacetat besteht.

6. Verwendung nach Anspruch 1,
wobei der Säuger ein Mensch ist.

7. Verwendung nach Anspruch 1,
wobei die mit einer Immunreaktion verbundene Erkrankung aus der Gruppe ausgewählt ist, die aus Kontaktdermatitis, allergischer Dermatitis, dem Sjogren-Syndrom, einschließlich der das Sjogren-Syndrom begleitenden Keratokonjunktivitis sicca, ekzematöser Dermatitis, Arznelmittel-Exanthemen, Lichen planus, Psoriasis, Alopecia areata, kutaner Lupus erythematosus, Sklerodermie, Asthma, allergischem Asthma, ulzerativer Kolitis, der Crohn-Krankheit, allergischen ulzerativen Kolitis-Reaktionen, die Reaktionen auf einen Arthropoden-Biß begleiten, aphthösen Geschwüren, Konjunktivitis, Iritis, Keratokonjunktivitis, Vaginitis und Proktitis besteht.

8. Verwendung nach Anspruch 1,
wobei das Medikament ein Mundwasser ist.

9. Verwendung nach Anspruch 1,
wobei das Medikament eine Lösung zum Gurgeln ist.

10. Verwendung nach Anspruch 1,
wobei die Verbindung in Kombination mit einem Ophthalmoträger vorliegt und das Medikament für die topische Anwendung beim Auge vorgesehen ist.

11. Verwendung nach Anspruch 1,
wobei das Medikament für die Anwendung auf der Haut vorgesehen ist.

12. Verwendung nach Anspruch 1, wobei das Medikament für die Anwendung auf Schleimhautmembranen vorgesehen ist.

13. Verwendung nach Anspruch 1,
wobei die Verbindung für die Anwendung in einer Konzentration von 100 mg/ml vorgesehen ist.

14. Verwendung nach Anspruch 1,
wobei die Verbindung in einer Konzentration von 0,001 bis 100 % im Medikament vorliegt.

15. Verwendung nach Anspruch 1,
wobei das Medikament eine Formulierung für eine zeitliche Freisetzung aufweist.

16. Verfahren nach Anspruch 1,
wobei das Medikament ein Retentionsklistier aufweist.

17. Verwendung nach Anspruch 1,
wobei das Medikament für die Verabreichung in Kombination mit einer Verbindung vorgesehen ist, die aus der Gruppe ausgewählt ist, die aus Antivirusmitteln, Antifungiziden, Antibiotika, entzündungshemmenden Mitteln und anderen Immunosuppressiva und Bronchodilatoren oder anderen therapeutischen Mitteln für Asthma besteht.

18. Verwendung einer Verbindung nach einem der Ansprüche 1 bis 5
ohne einen signifikanten neuroleptischen Effekt bei der Herstellung eines Medikamentes für die Behandlung eines Säugers, der einer Immunosuppression bedarf, wobei das Medikament für die systemische Verabreichung vorgesehen ist.

19. Verwendung nach Anspruch 18,
wobei der Säuger ein Mensch ist.

20. Verwendung nach Anspruch 18,
wobei der Säuger, der einer Immunosuppression bedarf, eine Krankheit hat, die aus der Gruppe ausgewählt ist, die aus Autoimmunkrankheiten, Krankheiten mit einer bekannten oder unbekannten Ätiologie mit einer immunologischen Komponente und Allergien besteht.

21. Verwendung nach Anspruch 20,
wobei die Autoimmunkrankheit aus der Gruppe ausgewählt ist, die aus rheumatischer Arthritis, juveniler rheumatischer Arthritis, psoriatischer Arthritis, Psoriasis, Lepra-Umkehrreaktionen, Erythema nodosum leprosum, Autoimmun-Uveitis, multipler Sklerose, allergischer Encephalomyelitis, systemischem Lupus erythematosis, akute Nekrose erzeugender, hämorrhagischer Encephalopathie, idiopathischem bilateralem fortschreitendem sensorineuralem Verlust des Gehörs, aplastischer Anämie, reiner Erythrocyten-Anämie, idiopathischer Thrombopenie, Polychondritis, Sklerodermie, der Wegner-Granulomatose, chronischer aktiver Hepatitis, Myasthenie gravis, atopischer Dermatitis, dem Stevens-Johnson-Syndrom, idiopathischer Psilose, der Crohn-Krankheit, ulzerativer Kolitis, der Graves-Ophthalmopathie, Sarkoidose, primärer biliärer Zirrhose, primärer juveniler Diabetes, Uveitis posterior und interstitieller Lungenfibrose besteht.

22. Verwendung nach Anspruch 18,
wobei das Buspiron oder das Buspiron-Derivat für die Verabreichung durch intravenöse Injektion vorgesehen ist.

23. Verwendung nach Anspruch 22,
wobei das Buspiron oder das Buspiron-Derivat für die orale Verabreichung vorgesehen ist.

24. Verwendung nach Anspruch 18,
wobei das Buspiron oder das Buspiron-Derivat oder dessen pharmazeutisch verträgliches Salz, außer einem quaternärem Salz, für die Verabreichung mittels eines transdermalen Pflasters vorgesehen ist.

25. Verwendung nach Anspruch 18,
wobei die Dosierung zwischen 20 und 0,0001 mg/kg Körpergewicht pro Tag als einzige tägliche Dosis oder als aufgeteilte tägliche Dosen liegt.

26. Verwendung nach Anspruch 18,
wobei das zu verabreichende Buspiron oder Buspiron-Derivat in einer Formulierung für eine zeitliche Freisetzung vorliegt.

27. Verwendung nach Anspruch 18,
wobei das zu verabreichende Buspiron oder dessen Derivat subkutan verabreicht wird.

28. Verwendung nach Anspruch 18,
wobei dem Patienten eine Verbindung oder Verbindungen verabreicht wird, wurde oder werden wird, die aus der Gruppe ausgewählt ist, die aus Antivirusmitteln, Antifungiziden, Antibiotika, entzündungshemmenden Mitteln und anderen Immunosuppressiva besteht.

29. Verwendung nach Anspruch 18,
wobei das Medikament ferner eine Verbindung oder Verbindungen aufweist, die aus der Gruppe ausgewählt ist bzw. sind, die aus Antivirusmitteln, Antifungiziden, Antibiotika, entzündungshemmenden Mitteln und anderen Immunosuppressiva besteht.

30. Pharmazeutische Zusammensetzung für die systemische Verabreichung, die ein Buspiron-Derivat aufweist, ohne einen signifikanten neuroleptischen Effekt, nach einem der Ansprüche 1 bis 5.

31. Zusammensetzung nach Anspruch 30 in einem topischen Träger in einer auf das Gewicht bezogenen Menge von 0,001 bis 100 % Buspiron-Derivat.

32. Zusammensetzung nach Anspruch 30,
wobei das Buspiron-Derivat Buspiron aufweist, das mit Cycloamylose in einem Komplex gebunden ist.

33. Zusammensetzung nach Anspruch 30,
wobei das Buspiron-Derivat in einer Formulierung für die zeitliche Freisetzung vorliegt.

34. Zusammensetzung nach Anspruch 30,
wobei das Buspiron-Derivat in Kombination mit einer Verbindung oder Verbindungen vorliegt, die aus der Gruppe ausgewählt ist bzw. sind, die aus Antivirusmitteln, Antifungiziden, Antibiotika, entzündungshemmenden Mitteln und anderen Immunosuppressiva besteht.

35. Pharmazeutische Zusammensetzung für die topische Verabreichung, die eine wirksame Menge eines Buspiron-Derivats nach einem der Ansprüche 1 bis 5 aufweist.

36. Zusammensetzung nach Anspruch 35 in einem topischen Träger in einer auf das Gewicht bezogenen Menge von 0,001 bis 100 % Buspiron-Derivat.

37. Verwendung nach Anspruch 25,
wobei die Dosierung zwischen 10 und 0,0001 mg/kg Körpergewicht pro Tag als einzige tägliche Dosis oder als aufgeteilte tägliche Dosen liegt.

## Revendications

1. Utilisation d'un composé choisi dans le groupe formé par la buspirone et un dérivé de buspirone de formule : 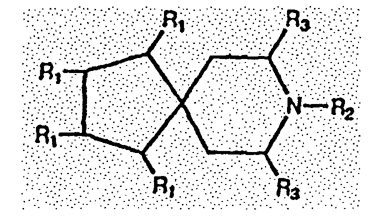 dans laquelle :
R₁ = H ; halogéno (chloro, bromo, fluoro ou iodo) ; alkyle, incluant spécifiquement CH₃-, cyclohexyle, (CH₃)₂CH-, CH₃(CH₂)₃-, (CH₃)₂CHCH₂-, CH₃CH₂CH(CH₃)-, (CH₃)₃C-, et CH₃(CH₂)ₚ- ; Y-CH₂(CH₂)ₙ- ; oxyalkyle ; C₆H₅-, aryle, (2, 3, ou 4)-(OCH₃)C₆H₄- et (2,3, ou 4)-(CH₃)C₆H₄- ; 2-X-C₆H₄-, 3-X-C₆H₄-, ou 4-X-C₆H₄- ; oxyaryle ; ou alkaryle ;
R₂ = H ; C₆H₅CH(CH₂CH₃)CH₂-, C₆H₅CH(CH₃)(CH₂)₂-, C₆H₅CH₂CH(CH₃)CH₂-, C₆H₅CH₂CH₃CH(CH₃)-, C₆H₅CH(CH₃)(CH₂)₃-, (2, 3, ou 4)-(alkyl)-C₆H₄CH(CH₃)(CH₂)₃-, (2, 3, ou 4)-(alkyloxy)-C₆H₄CH(CH₃)(CH₂)₃, (2, 3, ou 4)-X-C₆H₄-alkyle, (2, 3, ou 4)-X-C₆H₄CH(CH₂CH₃)CH₂-, (2, 3, ou 4)-X-C₆H₄CH(CH₃)(CH₂)-, 4-X-C₆H₄CH(CH₃)(CH₂)₂-, et 4-X-C₆H₄-CH(CH₃)(CH₂)₃- ; C₆H₅CH(OCH₃)(CH₂)₂-, 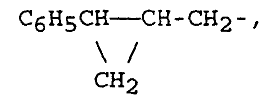 C₆H₅CO(CH₂)₃-, C₆H₅CO(CH₂)₄-, (2, 3, ou 4)-(alkyl)-C₆H₄CO(CH₂)₃-, (2, 3, ou 4)-(alkyloxy)-C₆H₄CO(CH₂)₃-, (2, 3, ou 4)-X-C₆H₄CO(CH₂)ₙ-, 2-thiényl-CO-(CH₂)₃-, -alkyl-pipérazinyl-aryle ; -alkyl-cycloalkyl en C₃₋₈-aryle ; -alkyl-pipérazinyl-hétérocycle ; -alkyl-cycloalkyl en C₃₋₈-hétérocycle ; -alkyl-cycloalkyl en C₃₋₈-Ar₁ ; -alkyl-pipérazinyl-Ar₁ ; -alcényl-pipérazinyl-aryle ; -alcényl-cycloalkyl en C₃₋₈-aryle ; -alkyl-aryl-hétérocycle ; -alkyl-hétérocycle-aryle ; -alcényl-cycloalkyl en C₃₋₈-Ar₁ ; -alcényl-pipérazinyl-hétérocycle ; -alcényl-cycloalkyl en C₃₋₈-hétérocycle ; -alcényl-pipérazinyl-Ar₁ ; 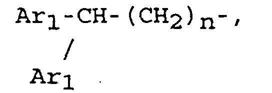 (2, 3, ou 4)-X-C₆H₄C(CH₃)CH(CH₂)₂-, où la conformation autour de la double liaison est cis ou trans, (2, 3, ou 4)-X-C₆H₄C(CH₃)CHCH₂-, où la conformation autour de la double liaison est cis ou trans, (2, 3, ou 4)-X-C₆H₄COCH=CHCH₂-, Y-CH₂(CH₂)ₙ-, Ar₁-(CH₂)ₙ-, alkyle en C₁ à C₂₀, X-(CH₂)ₙCO- ou X-(CH₂)ₙ- ;
R₃ = =O, =NH, =S, chloro, bromo, iodo, fluoro, alkyle, ou aryle ;
n = 1 à 6 ;
p = 1 à 20 ;
X = est indépendamment F, Cl, Br, I, OCH₃, SO₃⁻, NH₂, H, -OH, -COOH, -COOR, -SO₃H, -CN, -NHSO₃H, -NO₂ ou -SO₂NH₂ ;
Y = H, F, Cl, Br, I, -SO₃, -PO₄⁼, -OH, -SH, -SCH₃, -CH₃SO₂⁻, -NH₂ ou -CO₂⁻ ; et
Ar₁ = est indépendamment aryle, (2, 3, ou 4-X-C₆H₄-), (2, 3, ou 4) -(CH₂X)C₆H₄-, (2, 3, ou 4)-(CX₃)C₆H₄-, (2, 3, ou 4)-(CHX₂)C₆H₄-, 2-thiényle ou (2, 3, ou 4)-X-C₆H₄CH₂-,
ou son sel acceptable sur le plan pharmaceutique, incluant un sel quaternaire, dans la fabrication d'un médicament pour traiter une pathologie cutanée, oculaire ou d'une muqueuse associée à une réponse immunitaire chez l'homme ou un autre mammifère, dans laquelle le médicament est destiné à une application topique, dans laquelle
- alkyle désigne un groupe hydrocarboné en C₁ à C₂₀, saturé, droit, ramifié ou cyclique, incluant le méthyle, l'éthyle, le propyle, l'isopropyle, le butyle, l'isobutyle, le t-butyle, le pentyle, le cyclopentyle, l'isopentyle, le néopentyle, l'hexyle, l'isohexyle, le cyclohexyle, le 3-méthylpentyle, le 2,2-diméthylbutyle et le 2,3-diméthylbutyle, et dans laquelle
- aryle désigne un phényle ou phényle substitué, dans lequel le substituant est, indépendamment, un halogéno, alkyle, ou oxy(alkyle) (par exemple, méthoxy ou éthoxy) et dans laquelle l'aryle peut avoir jusqu'à trois substituants, et dans laquelle aralkyle désigne un groupe aryle avec un substituant alkyle.

2. Utilisation selon la revendication 1, dans laquelle le groupe alkyle est choisi dans le groupe formé par CH₃-, cyclohexyle, (CH₃)₂CH-, CH₃(CH₂)₃-, (CH₃)₂CHCH₂-, CH₃CH₂CH(CH₃)-, CH₃(CH₂)ₚ- et (CH₃)₃C-.

3. Utilisation selon la revendication 1, dans laquelle le groupe aryle est choisi dans le groupe formé par C₆H₅-, (2, 3, ou 4)-(OCH₃)C₆H₄- et (2, 3, ou 4)-(CH₃)C₆H₄- ; 2-X-C₆H₄-, 3-X-C₆H₄-, 4-X-C₆H₄-.

4. Utilisation selon la revendication 1, dans laquelle (2, 3, ou 4)-X-C₆H₄-alkyl- est choisi dans le groupe formé par (2, 3, ou 4)-X-C₆H₄CH(CH₂CH₃)CH₂-, (2, 3, ou 4)-X-C₆H₄CH(CH₃)(CH₂)-, (2, 3, ou 4)-X-C₆H₄CH(CH₃)(CH₂)₂-, et (2, 3 ou 4)-X-C₆H₄-CH(CH₃)(CH₂)₃-

5. Utilisation selon la revendication 1, dans laquelle le sel d'ammonium quaternaire est de formule -NR₃⁺Z⁻, dans laquelle R est un alkyle ou un benzyle, et Z est un contre-ion choisi dans le groupe formé par le chlorure, le bromure, l'iodure, le -O-alkyle, le toluènesulfonate, le méthylsulfonate, le sulfonate, le phosphate, le benzoate, le succinate, l'acétate, le glycolate, le succinate, le maléate, le malate, le citrate, le tartrate, l'ascorbate, le benzoate, le cinnamoate, le mandéloate, le benzyloate et l'acétate de diphényle.

6. Utilisation selon la revendication 1, dans laquelle le mammifère est un être humain.

7. Utilisation selon la revendication 1, dans laquelle la pathologie associée à une réponse immunitaire est choisie dans le groupe formé par l'eczéma de contact, la dermite atopique, le syndrome de Sjögren, incluant la kératoconjonctivite sèche secondaire au syndrome de Sjögren, la dermite eczémateuse, la toxidermie, le lichen plan, le psoriasis, la pelade, le lupus érythémateux cutané, la sclérodermie, l'asthme, l'asthme allergique, la rectocolite hémorragique, la maladie de Crohn, les réactions allergiques secondaires aux réactions de morsures d'arthropodes, l'ulcération aphteuse, la conjonctivite, l'iritis, la kératoconjonctivite, la vaginite et la rectite.

8. Utilisation selon la revendication 1, dans laquelle le médicament est un bain de bouche.

9. Utilisation selon la revendication 1, dans laquelle le médicament est un gargarisme.

10. Utilisation selon la revendication 1, dans laquelle le composé est en combinaison avec un véhicule ophtalmique et le médicament est destiné à une application topique pour l'oeil.

11. Utilisation selon la revendication 1, dans laquelle le médicament est destiné à une application cutanée.

12. Utilisation selon la revendication 1, dans laquelle le médicament est destiné à une application sur des membranes muqueuses.

13. Utilisation selon la revendication 1, dans laquelle le composé est destiné à une application à une concentration de 100 mg/mL.

14. Utilisation selon la revendication 1, dans laquelle le composé est présent dans le médicament à une concentration comprise entre 0,001% et 100%.

15. Utilisation selon la revendication 1, dans laquelle le médicament comprend une formulation à effet retard.

16. Procédé selon la revendication 1, dans lequel le médicament comprend un lavement médicamenteux.

17. Utilisation selon la revendication 1, dans laquelle le médicament est destiné à une administration en combinaison avec un composé choisi dans le groupe formé par les antiviraux, les antifongiques, les antibiotiques, les anti-inflammatoires et les autres immunosuppresseurs et bronchodilatateurs ou les autres agents thérapeutiques pour l'asthme.

18. Utilisation d'un composé selon l'une quelconque des revendications 1 à 5, sans effet neuroleptique significatif dans la fabrication d'un médicament pour traiter un mammifère nécessitant une immunosuppression, dans laquelle le médicament est destiné à une administration systémique.

19. Utilisation selon la revendication 18, dans laquelle le mammifère est un être humain.

20. Utilisation selon la revendication 18, dans laquelle le mammifère nécessitant une immunosuppression présente un trouble choisi dans le groupe formé par les maladies autoimmunes, les maladies à étiologie connue ou inconnue ayant une composante immunologique, et les allergies.

21. Utilisation selon la revendication 20, dans laquelle la maladie autoimmune est choisie dans le groupe formé par la polyarthrite rhumatoïde, la polyarthrite juvénile, le psoriasis arthropathique, le psoriasis, les réactions de réversion de la lèpre, erythème noueux lépreux, l'uvéite autoimmune, la sclérose en plaques, l'encéphalomyélite allergique, le lupus érythémateux disséminé, l'encéphalopathie hémorragique nécrotique aiguë, la surdité de perception progressive bilatérale idiopathique, l'anémie aplasique, l'érythroblastopénie, la thrombocytopénie idiopathique, la polychondrite, la sclérodermie, la granulomatose de Wegener, l'hépatite chronique active, la myasthénie gravis, la dermite atopique, le syndrome de Stevens-Johnson, la sprue idiopathique, la maladie de Crohn, la recto-colite hémorragique, l'ophtalmopathie de Graves, la sarcoidose, la cirrhose biliaire primitive, le diabète juvénile primitif, l'uvéite postérieure et la pneumopathie infiltrante diffuse.

22. Utilisation selon la revendication 18, dans laquelle la buspirone ou le dérivé de buspirone est destiné à une administration par injection intraveineuse.

23. Utilisation selon la revendication 22, dans laquelle la buspirone ou le dérivé de buspirone est destiné à une administration par voie orale.

24. Utilisation selon la revendication 18, dans laquelle la buspirone ou le dérivé de buspirone, ou son sel acceptable sur le plan pharmaceutique, autre qu'un sel quaternaire, est destiné à une administration par timbre transdermique.

25. Utilisation selon la revendication 18, dans laquelle le dosage est compris entre 20 mg/kg et 0,0001 mg/kg de poids corporel par jour sous forme de dose quotidienne unique ou de doses quotidiennes fractionnées.

26. Utilisation selon la revendication 18, dans laquelle la buspirone ou le dérivé de buspirone destiné à être administré est dans une formulation à effet retard.

27. Utilisation selon la revendication 18, dans laquelle la buspirone ou son dérivé est destiné à être administré par voie sous-cutanée.

28. Utilisation selon la revendication 18, dans laquelle le patient reçoit, a reçu ou recevra l'administration d'un ou de composés choisis dans le groupe formé par les antiviraux, les antifongiques, les antibiotiques, les anti-inflammatoires, et les autres immunosuppresseurs.

29. Utilisation selon la revendication 18, dans laquelle le médicament comprend de plus un ou des composés choisis dans le groupe formé par les antiviraux, les antifongiques, les antibiotiques, les anti-inflammatoires et les autres immunosuppresseurs.

30. Composition pharmaceutique pour une administration systémique comprenant un dérivé de buspirone sans effet neuroleptique significatif tel que défini dans l'une quelconque des revendications 1 à 5.

31. Composition selon la revendication 30, dans un véhicule topique en une quantité pondérale comprise entre 0,001 et 100% de dérivé de buspirone.

32. Composition selon la revendication 30, dans laquelle le dérivé de buspirone comprend la buspirone complexée avec une cycloamylose.

33. Composition selon la revendication 30, dans laquelle le dérivé de buspirone est dans une formulation à effet retard.

34. Composition selon la revendication 30, dans laquelle le dérivé de buspirone est en combinaison avec un composé ou des composés choisis dans le groupe formé par les antiviraux, les antifongiques, les antibiotiques, les anti-inflammatoires, et les autres immunosuppresseurs.

35. Composition pharmaceutique pour une délivrance topique comprenant une quantité efficace d'un dérivé de buspirone tel que défini dans l'une quelconque des revendications 1 à 5.

36. Composition selon la revendication 35, dans un véhicule topique en une quantité pondérale comprise entre 0,001 et 100% de dérivé de buspirone.

37. Utilisation selon la revendication 25, dans laquelle le dosage est compris entre 10 mg/kg et 0,0001 mg/kg de poids corporel par jour sous forme d'une dose quotidienne unique ou de doses quotidiennes fractionnées.
